(19) Europäisches Patentamt
European Patent Office
Office européen des brevets

(11) EP 0 573 878 B1

(12) EUROPÄISCHE PATENTSCHRIFT

(45) Veröffentlichungstag und Bekanntmachung des Hinweises auf die Patenterteilung:
**20.12.2000 Patentblatt 2000/51**

(51) Int. Cl.[7]: **C07D 213/61**, C07D 213/64, C07D 405/12, C09K 19/34, G09G 3/18, G02F 1/13

(21) Anmeldenummer: **93108777.9**

(22) Anmeldetag: **01.06.1993**

# (54) 3-Fluorpyridine, Verfahren zu ihrer Herstellung und ihre Verwendung in Flüssigkristallmischungen

3-fluoropyridines, process for their preparation and their use in liquid crystal mixtures

3-fluoropyridines, procédé pour leur préparation et leur utilisation dans des compositions de cristaux liquides

(84) Benannte Vertragsstaaten:
**AT BE CH DE DK ES FR GB GR IE IT LI LU MC NL PT SE**

(30) Priorität: **09.06.1992 DE 4218873**

(43) Veröffentlichungstag der Anmeldung:
**15.12.1993 Patentblatt 1993/50**

(73) Patentinhaber:
**Aventis Research & Technologies GmbH & Co. KG**
**65926 Frankfurt am Main (DE)**

(72) Erfinder:
- **Schlosser, Hubert, Dr.**
  **W-6246 Glashütten/Ts. (DE)**
- **Kaltbeitzel, Anke, Dr.**
  **W-6090 Rüsselsheim (DE)**

(74) Vertreter:
**Isenbruck, Günter, Dr.**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

(56) Entgegenhaltungen:
**EP-A- 0 206 228**
**EP-A- 0 284 093**
**EP-A- 0 475 444**
**WO-A-91/04248**
**WO-A-91/04249**
**WO-A-92/09576**
**WO-A-92/11241**

- **DATABASE WPI Week 9032, Derwent Publications Ltd., London, GB; AN 90-242916 & JP-A-2 169 537 (KANTO KAGAKU K.K.)**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7 (HRS)/3.6

## Beschreibung

Die ungewöhnliche Kombination von anisotropem und fluidem Verhalten der Flüssigkristalle hat zu ihrer Verwendung in elektrooptischen Schalt- und Anzeigevorrichtungen geführt. Dabei können ihre elektrischen, magnetischen, elastischen und/oder thermischen Eigenschaften zu Orientierungsänderungen genutzt werden. Optische Effekte lassen sich beispielsweise mit Hilfe der Doppelbrechung, der Einlagerung dichroitisch absorbierender Farbstoffmoleküle ("guest-host mode") oder der Lichtstreuung erzielen.

Zur Erfüllung der ständig steigenden Praxisanforderungen auf den verschiedenen Anwendungsgebieten besteht laufend ein Bedarf an neuen verbesserten Flüssigkristall ("liquid crystal")-Mischungen und somit auch an einer Vielzahl mesogener Verbindungen unterschiedlicher Struktur. Dies gilt sowohl für die Gebiete, bei denen nematische LC-Phasen verwendet werden, als auch für solche mit smektischen LC-Phasen.

Auf besonderes Interesse sind in den letzten Jahren ferroelektrische flüssigkristalline Mischungen (FLC-Mischungen) gestoßen (siehe z. B. J.W. Goodby, Ferroelectric Liquid Crystals, Gordon and Breach, Philadelphia 1991). Für die praktische Verwendung von ferroelektrischen Flüssigkristallen in elektrooptischen Anzeigen werden chirale, geneigt-smektische Phasen, wie $S_C^*$ -Phasen benötigt [siehe z.B. R.B. Meyer, L.Liebert, L. Strzelecki und P. Keller, J. Physique <u>36</u>, L-69 (1975)], die über einen großen Temperaturbereich stabil sind. Dieses Ziel kann man mit Verbindungen erreichen, die selbst solche Phasen, z. B. $S_C^*$ -Phasen, ausbilden, oder aber indem man nicht chirale geneigt-smektische Phasen ausbildende Verbindungen mit optisch aktiven Verbindungen dotiert [siehe z.B. M. Brunet, Cl. Williams, Ann. Phys. <u>3</u>, 237 (1978)].

Bei der Verwendung ferroelektrischer Flüssigkristallmischungen in elektrooptischen Bauelementen ist eine einheitliche planare Orientierung der Flüssigkristalle notwendig, um ein hohes Kontrastverhältnis zu erzielen. Es hat sich gezeigt, daß sich eine einheitliche planare Orientierung in der $S_C^*$ -Phase erreichen läßt, wenn die Phasenfolge der Flüssigkristallmischung mit abnehmender Temperatur lautet: Isotrop→nematisch→smektisch A→smektisch C (siehe z.B. K. Flatischler et al., Mol. Cryst. Liq. Cryst. 131, 21 (1985); T. Matsumoto et al., p. 468 - 470, Proc. of the 6th Int. Display Research Conf., Japan Display, 30. September - 2. October 1986, Tokyo, Japan; M. Murakami et al., ibid, p. 344 - 347).

Für ferroelektrische (chiral smektische) Flüssigkristallmischungen muß zusätzlich die Bedingung erfüllt sein, daß die Ganghöhe (pitch) der Helix in der $S^*_C$-Phase groß, d. h. größer als 5 µm, und in der N*-Phase sehr groß, d. h. größer als 10 µm, bzw. unendlich ist.

Die optische Schaltzeit $\tau$[µs] ferroelektrischer Flüssigkristallsysteme, die möglichst kurz sein soll, hängt von der Rotationsviskosität des Systems $\gamma$[mPas], der spontanen Polarisation $P_S$[nC/cm$^2$] und der elektrischen Feldstärke E[V/m] ab nach der Beziehung

$$\tau \approx \frac{\gamma}{P_S \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$, von vorzugsweise $\approx$ O,13, und eine geringe positive oder vorzugsweise negative dielektrische Anisotropie $\Delta\epsilon$ verlangt (siehe z.B. S. T. Lagerwall et al., "Ferroelectric Liquid Crystals for Displays" SID Symposium, Oct. Meeting 1985, San Diego, Ca, USA).

Die Gesamtheit dieser Forderungen ist im allgemeinen nur mit Mischungen aus mehreren Komponenten zu erfüllen. Als Basis (oder Matrix) dienen dabei bevorzugt Verbindungen, die selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Einzelne dieser Komponenten und auch bestimmte Mischungen sind bereits aus dem Stand der Technik bekannt. Da aber die Entwicklung, insbesondere von ferroelektrischen Flüssigkristallmischungen, noch in keiner Weise als abgeschlossen betrachtet werden kann, sind die Hersteller von Displays an unterschiedlichen Mischungen interessiert. Dieses ist u. a. auch deshalb so, weil erst das Zusammenwirken der flüssigkristallinen Mischungen mit den einzelnen Bauteilen der Anzeigevorrichtungen bzw. der Zellen (z. B. der Orientierungsschicht) Rückschlüsse auf die Qualität auch der flüssigkristallinen Mischungen zuläßt.

In EP-A 0 158 137 werden 4-Fluorpyrimidine als Verbindungen und als Mischungskomponenten allgemein beschrieben. Sie weisen jedoch keine oder nur eine geringe Tendenz zur Ausbildung smektischer Phasen auf und finden daher Einsatz in nematischen Mischungen.

In EP-A 0 284 093 werden Pyridine beschrieben, die beim Zusatz von nematischen Flüssigkristallzusammensetzungen die Temparaturabhängigkeit der Schwellspannung herabsetzen können.

In DE-A 40 29 165 sowie in DE-A 40 30 582 werden 4-Fluorpyrimidine als Komponenten für ferroelektrische Flüssigkristallmischungen vorgestellt.

Desweiteren ist bekannt, daß Mono- und Difluorphenylverbindungen als Komponenten von Flüssigkristallmischungen verwendet werden können (siehe z.B. JP-A 2169-537; V.Reiffenrath, The Twelfth International Liquid Crystal Conference, Freiburg, 15.-19.August 1988). Diese Verbindungen haben jedoch zum Teil keine $S_C$-Phase.Ferner treten aufgrund fluorophober Wechselwirkungen häufig Mischbarkeitsprobleme mit strukturell unterschiedlichen Mischungskomponenten, z. B. Phenylpyrimidinen, auf.

Pyridinderivate zeigen ebenfalls flüssigkristallines Verhalten unter Ausbildung einer $S_C$-Phase (T.Geelhaar, 1st International Symposium on Ferroelectric Liquid Crystals, Arcachon, 2. - 23. September 1987; US 4,952,335). Eine in diesen Verbindungen häufig auftretende $S_I$-Phase beeinträchtigt jedoch deren Verwendung in ferroelektrischen Flüssigkristallmischungen.

Derivate des 2-Fluorpyridins mit ausschließlich nematischen Phasen zur Verwendung in Flüssigkristallmischungen werden in WO-91 /04249 und WO-91/04248 beschrieben.

Gegenstand der vorliegenden Erfindung sind 3-Fluorpyridin-Derivate der Formel (I) und deren Verwendung als Komponenten für ferroelektrische Flüssigkristallmischungen, wobei mindestens ein 3-Fluorpyridin der allgemeinen Formel (I) als Komponente in einer Flüssigkristallmischung eingesetzt wird.

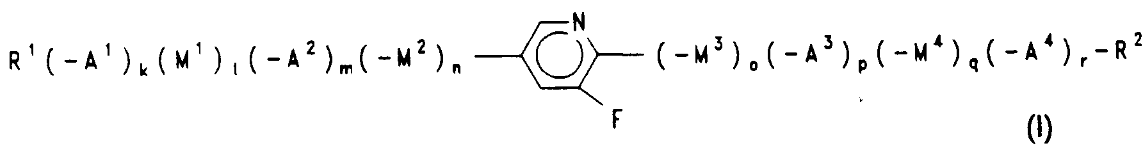

(I)

Die Symbole haben hierbei folgende Bedeutung:

$R^1$, $R^2$ gleich oder verschieden, -H, -F, oder geradkettiges oder verzweigtes (mit oder ohne asymmetrisches C-Atom)Alkyl mit 1 bis 16 C-Atomen, wobei auch eine oder zwei nicht benachbarte $-CH_2$-Gruppen durch -O-,-CO-,-CO-O-,-O-CO-, -O-CO-O-, -CH=CH-,-C≡C-,

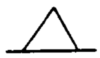

oder $-Si(CH_3)_2$- ersetzt sein können, und wobei auch ein oder mehrere H-Atome des Alkylrestes durch -F, substituiert sein können, oder die nachfolgende chirale Gruppe:

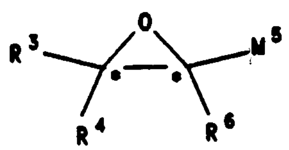

.

$A^1$, $A^2$, $A^3$, $A^4$ gleich oder verschieden 1-4-Phenylen, Pyridin-2,5-diyl, Pyrimidin-2,5-diyl, wobei ein oder zwei H-Atome durch F ersetzt sein können, trans-1,4-Cyclohexylen, Naphthalin-2,6-diyl,

$M^1$, $M^2$, $M^3$, $M^4$ gleich oder verschieden -CO-O, -O-CO-, $-CH_2$-O-, -O-$CH_2$-,

$R^3$, $R^4$, $R^6$, $R^7$ gleich oder verschieden, H oder geradkettiges oder verzweigtes Alkyl mit 1 bis 16 bzw. 3 bis 16 C-Atomen,

$M^5$ $-CH_2$-O-, -CO-O-, $-O-OH_2$-, -O-CO-k, l, m, n, o, p, q, r Null oder Eins, unter der Bedingung, daß die Summe k+m+p+r kleiner 4 und größer Null ist

In einer bevorzugten Ausführung der Erfindung haben die Symbole in der Formel (I) folgende Bedeutung:

$R^1$, $R^2$ gleich oder verschieden -H, -F oder geradkettiges oder verzweigtes (mit oder ohne asymmetrisches C-Atom) Alkyl mit 1 bis 16 C-Atomen, wobei auch eine $-CH_2$-Gruppe durch -O-, -CO-, -CO-O-, -O-CO-, -O-CO-O-,

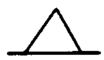

oder $-Si(CH_3)_2$- ersetzt sein kann, oder die nachfolgende chirale Gruppen:

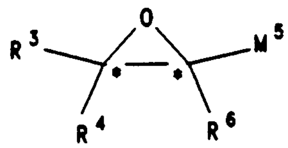

$A^1$, $A^2$, $A^3$, $A^4$ gleich oder verschieden, 1,4-Phenylen, Pyridin-2,5-diyl, Pyrimidin-2,5-diyl, wobei ein oder zwei H-Atome jeweils durch F ersetzt sein können, trans-1,4-Cyclohexylen, Naphthalin-2,6-diyl,
$M^1$, $M^2$, $M^3$, $M^4$ gleich oder verschieden, -CO-O-, -O-CO-, $-CH_2$-O-, $-O-CH_2$-,
$R^3$, $R^4$, $R^6$, $R^7$ gleich oder verschieden H oder geradkettiges oder verzweigtes Alkyl mit 1 bis 10 bzw. 3 bis 10 C-Atomen,
$M^5$ $-CH_2$-O-, -CO-O-, $-O-CH_2$-, -O-CO-

Ferner werden 3-Fluorpyridin-Derivate der Formel (I) bevorzugt, bei denen die Symbole folgende Bedeutung haben:

$R^1$, $R^2$ gleich oder verschieden, -H, -F, oder geradkettiges oder verzweigtes (mit oder ohne asymmetrisches C-Atom) Alkyl mit 1 bis 16 C-Atomen, wobei auch eine $-CH_2$-Gruppe durch -O-,-CO-, -CO-O-, -O-CO-, oder $-Si(CH_3)_2$- ersetzt sein kann, oder eine die nachfolgende chirale Gruppen:

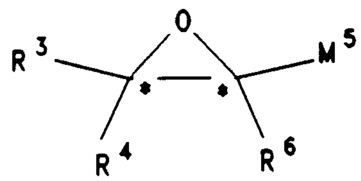

$A^1$, $A^2$, $A^3$, $A^4$ gleich oder verschieden, 1,4-Phenylen, Pyridin-2,5-diyl, Pyrimidin-2,5-diyl, bei dem ein oder zwei H-Atome durch F ersetzt sein können, trans-1,4-Cyclohexylen, Naphthalin-2,6-diyl

$M^1$, $M^2$, $M^3$, $M^4$ gleich oder verschieden -CO-O-, -O-CO-, $-O-CH_2$-, $-CH_2$-O-,

$R^3$, $R^4$, $R^6$, $R^7$ gleich oder verschieden H oder geradkettiges oder verzweigtes Alkyl mit 1 bis 10 C-Atomen, -

$M^5$ $-CH_2$-O-, -CO-O-, $-O-CH_2$-, -O-CO- .

Insbesondere bevorzugt ist ein 3-Fluorpyridin der Formel (I), bei dem $R^1$, $R^2$ gleich oder verschieden H oder Alkyl mit 1 bis 16 C-Atomen ist, wobei auch eine - $CH_2$-Gruppe durch -O-, -CO-O- oder -O-CO- ersetzt sein kann, oder die chirale Gruppe

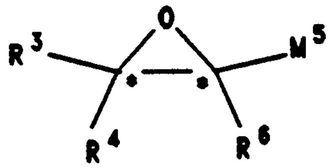

$A^1$, $A^2$, $A^3$, $A^4$ gleich oder verschieden, 1,4-Phenylen, Pyridin-2,5-diyl, Pyrimidin-2,5-diyl, bei dem ein oder zwei H-Atome jeweils durch F ersetzt sein können, trans-1,4-Cyclohexylen, Naphthalin-2,6-diyl;

$M^1$, $M^2$, $M^3$, $M^4$ sind gleich oder verschieden, -CO-O-, -O-CO-, -$OCH_2$--$CH_2$-O- ;

$R^3$, $R^4$, $R^6$ gleich oder verschieden H oder ein geradkettiges Alkyl mit 1 bis 10 C-Atomen;

$M^5$ -$CH_2$-O- oder -CO-O- ist.

Die erfindungsgemäßen Verbindungen sind chemisch und photochemisch stabil. Sie verfügen über niedrige Schmelzpunkte und im allgemeinen breite flüssigkristalline Phasen, insbesondere breite nematische, smektische A und smektische C-Phasen.
Weiterhin zeichnen sich die erfindungsgemäßen Verbindungen durch eine erhöhte Mischbarkeit, niedrige Viskosität und $\Delta$ $\varepsilon$-Werte die nahe bei Null liegen aus.

Aus flüssigkristallinen Verbindungen mit diesem Strukturelement lassen sich sowohl ferroelektrische Mischungen als auch nematische oder auch chiral nematische Mischungen herstellen, die für die Anwendung in elektrooptischen oder vollständig optischen Elementen, z. B. Anzeigeelementen, Schaltelementen, Lichtmodulatoren Elementen zur Bildbearbeitung, Signalverarbeitung oder allgemein im Bereich der nichtlinearen Optik geeignet sind.

Die erfindungsgemäßen Flüssigkristallmischungen bestehen im allgemeinen aus 2 bis 20, vorzugsweise 2 bis 15 Komponenten, darunter mindestens eine Verbindung der Formel (I). Die anderen Bestandteile werden vorzugsweise ausgewählt aus den bekannten Verbindungen mit nematischen, cholesterischen und/oder geneigtsmektischen Phasen, dazu gehörten beispielsweise Schiffsche Basen, Biphenyle, Pyridine, Thiadiazole, Difluorphenyle, Terphenyle, Phenylcyclohexane, Cyclohexylbiphenyle, Pyrimidine, Zimtsäureester, Cholesterinester sowie mehrkernige Ester von p-Alkylbenzoesäuren. Im allgemeinen liegen die im Handel erhältlichen Flüssigkristallmischungen bereits vor der Zugabe der erfindungsgemäßen Verbindung(en) als Gemische verschiedener Komponenten vor, von denen mindestens eine mesogen ist.

Von dem oder den erfindungsgemäßen 3-Fluorpyridin-Derivaten enthalten die Flüssigkristallmischungen im allgemeinen 0,1 bis 70 Mol-%, bevorzugt 0,5 bis 50 Mol-%, insbesondere 1 bis 25 Mol-%.

Für die gebrauchsfertigen ferroelektrischen Flüssigkristallmischungen wurden die Werte für die spontane Polarisation $P_s$[nC/cm$^2$], den Kontrast K und die optische Schaltzeit $\tau$[$\mu$s] bestimmt, wobei alle Messungen bei einer Temperatur von 25 °C vorgenommen wurden.

Die $P_s$-Werte werden nach der Methode von H. Diamant et al. (Rev. Sci. Instr., 28, 30, 1957) gemessen, wobei Meßzellen mit 2 $\mu$m Elektrodenabstand und geriebenem Polyimid als Orientierungsschicht verwendet werden.
Zur Bestimmung von $\tau$ und K wird die Meßzelle auf dem Drehtisch eines Polarisationsmikroskops zwischen gekreuztem Analysator und Polarisator befestigt. Für die Bestimmung des Kontrastes (K) wird die Meßzelle durch Drehen so positioniert, daß eine Photodiode minimalen Lichtdurchgang anzeigt (Dunkelzustand). Die Mikroskop-Beleuchtung wird so geregelt, daß die Photodiode für alle Zellen die gleiche Lichtintensität anzeigt. Nach einem Schaltvorgang ändert sich die Lichtintensität (Hellzustand) und der Kontrast wird aus dem Verhältnis der Lichtintensitäten dieser Zustände berechnet.

Zur Bestimmung von $\tau$ und des Schaltwinkels $\phi_{eff}$ wird durch Drehen des Tisches die Position des Tisches mit minimalem Lichtdurchgang für die beiden Schaltzustände in der Zelle bestimmt. Die Differenz der beiden Positionen am Drehtisch ist gleich dem doppelten effektiven Tiltwinkel. Mit Hilfe einer Photodiode erfolgt die Bestimmung der Schaltzeit $\tau$, indem die Anstiegszeit des Lichtsignals von 10 auf 90 % Signalhöhe gemessen wird. Die Schattspannung besteht aus Rechteckpulsen und beträgt $\pm$ 10 V/$\mu$m.

Die Phasenumwandlungstemperaturen werden beim Aufheizen mit Hilfe eines Polarisationsmikroskops anhand der Texturänderungen bestimmt. Die Bestimmung des Schmelzpunkts wurde hingegen mit einem DSC-Gerät durchgeführt. Die Angabe der Phasenumwandlungstemperaturen zwischen den Phasen

| | |
|---|---|
| Nematisch | (N bzw. N*) |
| Smektisch-C | ($S_C$ bzw. $S_C$*) |
| Smektisch-A | ($S_A$ bzw. $S_A$*) |
| Kristallin | (X) |

erfolgt in °C und die Werte stehen zwischen den Phasenbezeichnungen in der Phasenfolge.

Flüssigkristalline Mischungen, die Verbindungen der allgemeinen Formel (I) enthalten, sind besonders für die Verwendung in elektrooptischen Schalt-und Anzeigevorrichtungen (Displays) geeignet. Schalt- und Anzeigevorrichtungen (LC-Displays) weisen u. a. folgende Bestandteile auf: ein flüssigkristallines Medium, Trägerplatten (z. B. aus Glas oder Kunststoff), beschichtet mit transparenten Elektroden, mindestens eine Orientierungsschicht, Abstandshalter, Kleberahmen, Polarisatoren sowie für Farbdisplays dünne Farbfilterschichten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (z. B. E. Kaneko, "Liquid Crystal TV Displays: Principles and Applications of Liquid Crystal Displays", KTK Scientific Publishers, 1987, Seiten 12 - 30 und 63 - 172).

Die Herstellung der erfindungsgemäßen Verbindungen kann beispielsweise durch das in den Schemata 1 bis 6 skizzierte Verfahren erfolgen, bei dem über die Zwischenstufe 2,5-Dibrom-3-fluorpyridin (VI) durch mehrstufige Umsetzung die Seitenketten
$R^1(-A^1)_k(-M^1)_l(-A^2)_m(-M^2)_n$- und $(-M^3)_o(-A^3)_p(-M^4)_q(-A^4)_r$-$R^2$ in die 2- bzw. 5-Position des Pyridinrings eingebracht werden.

Ausgangsverbindung des erfindungsgemäßen Herstellungsverfahrens ist das kommerziell erhältliche 2-Amino-5-brompyridin (II), welches analog der von T. S. Safonova und L. G. Levkovskaya in Khim. Geterotsikl. Soedin 1968, Seite 997 - 1000 beschriebenen Methode mit Salpeter- und Schwefelsäure in 5-Brom-2-hydroxy-3-nitropyridin (III) überführt werden kann.

Durch Umsetzung des 2-Hydroxypyridins (III) mit einem Bromierungsmittel, wie Phosphortribromid, Phosphoroxytribromid und Phosphorpentabromid, bei Temperaturen zwischen 50 und 200 °C, insbesondere zwischen 100 und 150 °C, wird das 2,5-Dibrom-3-nitropyridin (IV) erhalten, welches nach ansich literaturbekannten Methoden (siehe z. B. Houben-Weyl, Methoden der Organischen Chemie, Georg Thieme Verlag, Stuttgart) zum 3-Amino-2,5-dibrompyridin (V) reduziert wird.

Verbindung (V) läßt sich nach der Methode von Balz und Schiemann (siehe Houben-Weyl, Methoden der Organischen Chemie, Georg Thieme Verlag, Stuttgart) über die Diazotierung zum Diazonium-tetrafluoroborat und die thermische Zersetzung desselben in 2,5-Dibrom-3-fluoropyridin (VI) umwandeln.

Eine alternative Ausgangsverbindung des erfindungsgemäßen Herstellungsverfahrens ist 3-Fluor-2-hydroxypyridin (XIV), welches nach der von M.P. Cava und B. Weinstein im Journal of Organic Chemistry 23 (1958) auf den Seiten 1616 bis 1617 beschriebenen Methode hergestellt werden kann.

Durch Umsetzung von 3-Fluor-2-hydroxypyridin (XIV) mit Brom bei Temperaturen zwischen -70°C und 150°C, insbesondere zwischen -20°C und 50°C in einem inerten Lösungsmittel wird 5-Brom-3-fluor-2-hydroxypyridin (XV) erhalten, welches durch Behandlung mit einem Bromierungsmittel, wie Phosphortribromid, Phosphoroxytribromid und Phosphorpentabromid, bei Temperaturen zwischen 50°C und 250°C, insbesondere zwischen 100°C und 170°C in das 2,5-Dibrom-3-fluorpyridin (VI) überführt werden kann.

Der Austausch des Brom-Substituenten in 2-Position der Verbindung (VI) gegen eine Gruppierung der allgemeinen Formel $Z^1 = (-M^3)_o(-A^3)_p(-M^4)_q(-A^4)_r$-$R^2$ durch Umsetzung mit einer Metallverbindung von $Z^1$, z. B. einer Lithium-, Natrium-, Kalium- oder Magnesiumverbindung, bei Temperaturen zwischen -40 und 100 °C, insbesondere zwischen -10 und 70 °C, in einem inerten Reaktionsmedium, z. B. Diethylether, Tetrahydrofuran, 1,4-Dioxan, Ethylenglykoldiethylether oder Diethylenglykoldiethylether, führt zu Verbindungen der Formel (VII).

Die Kreuzkupplung von Verbindung (VI) mit metallorganischen Derivaten von $Z^1$, z. B. Grignard-, Lithium- und Zinkderivaten, sowie Boronsäuren von $Z^1$ unter Verwendung von Übergangsmetallkatalysatoren, z. B. [1,3-bis(diphenylphosphino)-propan]nickel(II)chlorid und Tetrakis(triphenylphosphin)palladium(0) bei Temperaturen zwischen -40 und 200 °C, insbesondere zwischen -10 und 100 °C, in Reaktionsmedien, wie Benzol/Ethanol/Wasser für die Umsetzung mit Boronsäuren von $Z^1$ und z. B. Diethylether oder Tetrahydrofuran für die Umsetzung mit Grignard-, Lithium- und Zinkderivaten von $Z^1$, liefert ebenfalls Verbindungen des Typs (VII).

Durch Kreuzkupplung von Verbindungen des Typs (VII) mit metallorganischen Derivaten von $Z^2$, z. B. Grignard-, Lithium- und Zinkderivaten, sowie Boronsäuren von $Z^2$ unter Verwendung von Übergangsmetallkatalysatoren, z. B. [1,3-bis(diphenylphosphino)propan]nickel(II)chlorid und Tetrakis(triphenylphosphin)palladium(0) bei Temperaturen zwischen -40 und 200 °C, insbesondere zwischen -10 und 100 °C, in Reaktionsmedien, wie Benzol/Ethanol/Wasser für die Umsetzung mit Boronsäuren von $Z^2$ und Diethylether oder Tetrahydrofuran für die Umsetzung mit Grignard-, Lithium- und Zinkderivaten von $Z^2$, erhält man 3-Fluorpyridine (I).

3-Fluorpyridine des Typs (VII) können durch Behandlung mit einem Lithiumalkyl, wie n-Butyllithium, tert.-Butyllithium oder Methyllithium, bei Temperaturen zwischen 100 und 50 °C, insbesondere zwischen -80 und 10 °C, in einem inerten Reaktionsmedium, z. B. Diethylether, Tetrahydrofuran oder Ethylenglykoldimethylether, in 3-Fluor-5-lithiumpyridine der Formel (VIII) überführt werden. 5-Lithiumpyridine der allgemeinen Formel (VIII) sind der Umsetzung mit elektrophilen Verbindungen zugänglich, wodurch entweder direkt oder über weitere Zwischenstufen (Verbindungen (IX), (X), (XI), (XII) und (XIII)) 3-Fluorpyridine der Formel (I) erhalten werden können.

So führen 3-Fluor-5-lithiumpyridine (VIII) nach der Behandlung mit Kohlendioxid bei Temperaturen zwischen -100 und 50 °C, insbesondere zwischen -80 und 10 °C, in einem inerten Reaktionsmedium, z. B. Diethylether, Tetrahydrofuran oder Ethylenglykoldiethylether zu 3-Fluor-5-pyridincarbonsäuren der allgemeinen Formel (IX). Die Spezies (IX) können nach an sich literaturbekannten Methoden (siehe z. B. Houben-Weyl, Methoden der Organischen Chemie, Georg Thieme Verlag, Stuttgart), entweder direkt durch Veresterung mit Alkoholen von $Z^3$ unter Zuhilfenahme geeigneter Kondensationsmittel, z. B. Carbodiimiden, zu 3-Fluopyridinen (I) oder nach Reduktion zu 3-Fluor-5-hydroxymethylpyridinen (X) mit geeigneten Reduktionsmitteln, z. B. komplexen Hydriden, durch Veresterung mit Carbonsäuren bzw. Carbonsäurehalogeniden von $Z^3$ oder durch Veretherung mit Alkoholen bzw. Halogeniden von $Z^3$ zu Verbindungen der Formel (I) umgesetzt werden.

Die Reaktion von Verbindungen des Typs (VIII) mit Nitrilen, Carbonsäurehalogeniden und Formylmethylderivaten von $Z^3$ bei Temperaturen zwischen -100 und 50 °C, insbesondere zwischen -80 und 10 °C, in einem inerten Reaktionsmedium, z. B. Diethylether, Tetrahydrofuran oder Ethylenglykoldiethylether, führt direkt zu 3-Fluorpyridinen der Formel (I). Olefinische 3-Fluorpyridine (I) lassen sich durch Hydrierung der olefinischen Doppelbindung nach an sich literaturbekannten Methoden (siehe z. B. Houben-Weyl, Methoden der Organischen Chemie, Georg Thieme Verlag, Stuttgart) in gesättigte Spezies (I) umwandeln.

Durch Reaktion von 3-Fluor-5-lithiumpyridinen (VIII) mit Ameisensäureamiden bei Temperaturen zwischen -100 und 50 °C, insbesondere zwischen -80 und 10 °C, in einem inerten Rekationsmedium, z. B. Diethylether, Tetrahydrofuran oder Ethylenglykoldiethylether werden 3-Fluor-5-formylpyridine (XI) erhalten, welche nach der sauer katalysierten Acetalisierung mit 2-$Z^4$-1,3-Propandiolen nach an sich literaturbekannten Methoden (siehe z. B. Houben-Weyl, Methoden der Organischen Chemie, Georg Thieme Verlag, Stuttgart) 3-Fluorpyridine des Typs (I) liefern.

Bei der sukzessiven Behandlung der 3-Fluor-lithiumpyridine (VII) mit Borsäuretrialkylestern bei Temperaturen zwischen -100 und 50 °C, insbesondere zwischen -80 und 10 °C, und wäßriger Säure bei Temperaturen zwischen -10 und 50 °C, insbesondere zwischen 10 und 30 °C, in einem inerten Reaktionsmedium, z. B. Diethylether, Tetrahydrofuran oder Ethylenglykoldiethylether, werden 3-Fluor-5-pyridinboronsäuren der Formel (XII) erhalten.

Die Boronsäuren (XII) können Kupplungsreaktionen mit Halogeniden von $Z^3$ unter Verwendung eines Übergangsmetallkatalysators, z. B. Tetrakis(triphenylphosphin)palladium[0], bei Temperaturen zwischen 30 und 200 °C, insbesondere zwischen 50 und 100 °C, in Reaktionsmedien, wie Benzol/Ethanol/Waser, zur Darstellung von Verbindungen des Typs (I) unterworfen werden.

3-Fluorpyridine (I) werden aus den Boronsäuren (XII) desweiteren durch deren Veresterung mit 2-$Z^4$-1,3-Propandiolen nach an sich literaturbekannten Methoden (siehe z. B. Houben-Weyl, Methoden der Organischen Chemie, Georg Thieme Verlag, Stuttgart) erhalten.

Die Oxidation der Boronsäuren (XII) mit Peroxiden, z. B. Wasserstoffperoxid, bei Temperaturen zwischen 10 und 100 °C, insbesondere zwischen 30 und 70 °C, in Reaktionsmedien, wie z. B. Diethylether oder Tetrahydrofuran, führt zu den 3-Fluor-5-hydroxypyridinen (XIII), welche sich nach an sich literaturbekannten Methoden (siehe z. B. Houben-Weyl, Methoden der Organischen Chemie, Georg Thieme Verlag, Stuttgart) durch Veresterung mit Carbonsäuren bzw. Carbonsäurehalogeniden von $Z^3$ oder durch Veretherung mit Alkoholen bzw. Halogeniden von $Z^3$ in 3-Fluorpyridine der allgemeinen Formel (I) überführen lassen.

Schema 1:

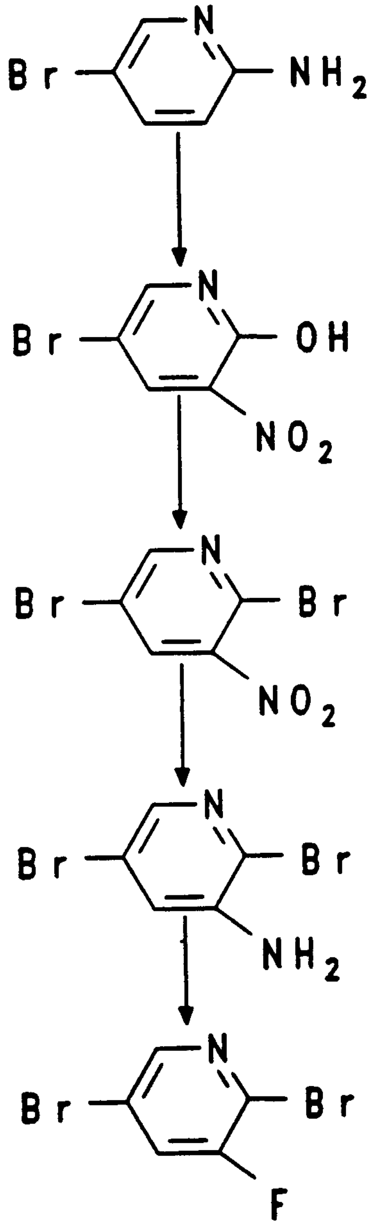

(II)

$HNO_3, H_2SO_4$

(III)

Bromierung

(IV)

Reduktion

(V)

1. $H^+, NaNO_2, HBF_4$

2. $\Delta T$

(VI)

**Schema 2:**

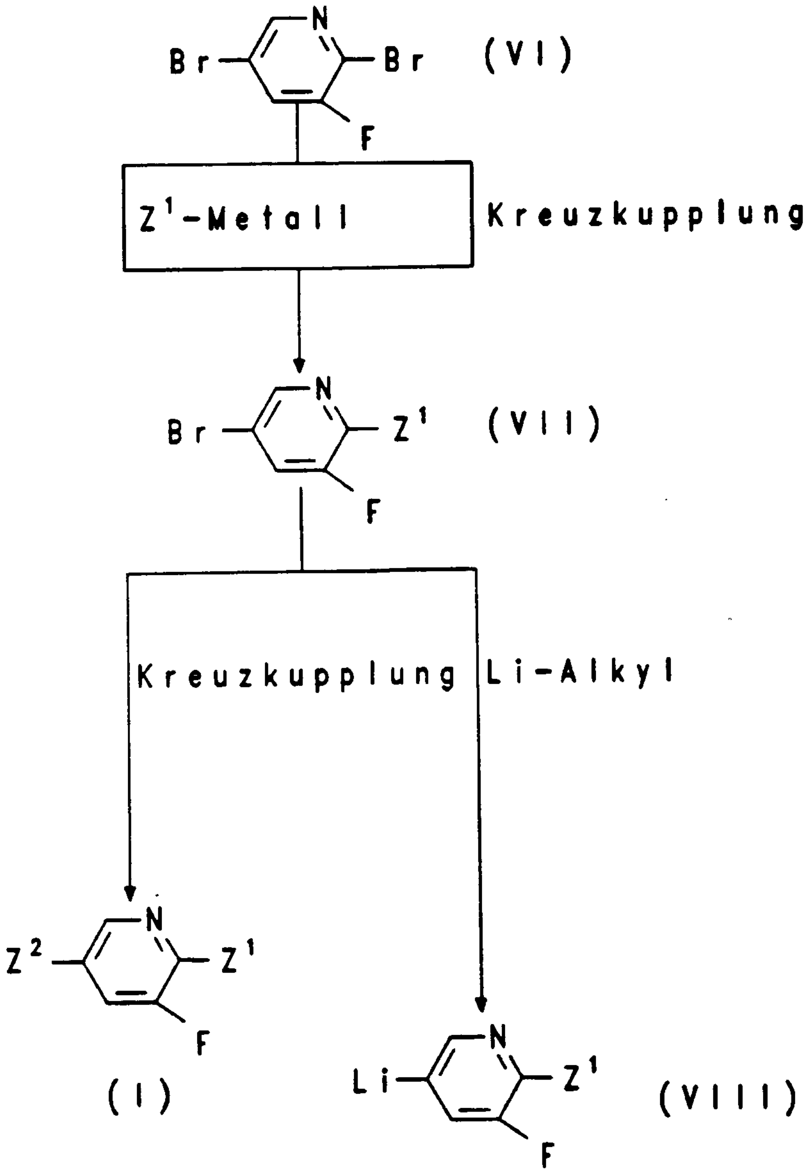


(VI)

(VII)

(I)

(VIII)

$Z^1 = (-M^3)_o(-A^3)_p(-M^4)_q(-A^4)_r-R^2$
$Z^2 = R^1(-A^1)_k(-M^1)_l(-A^2)_m(-M^2)_n-$

Schema 3:

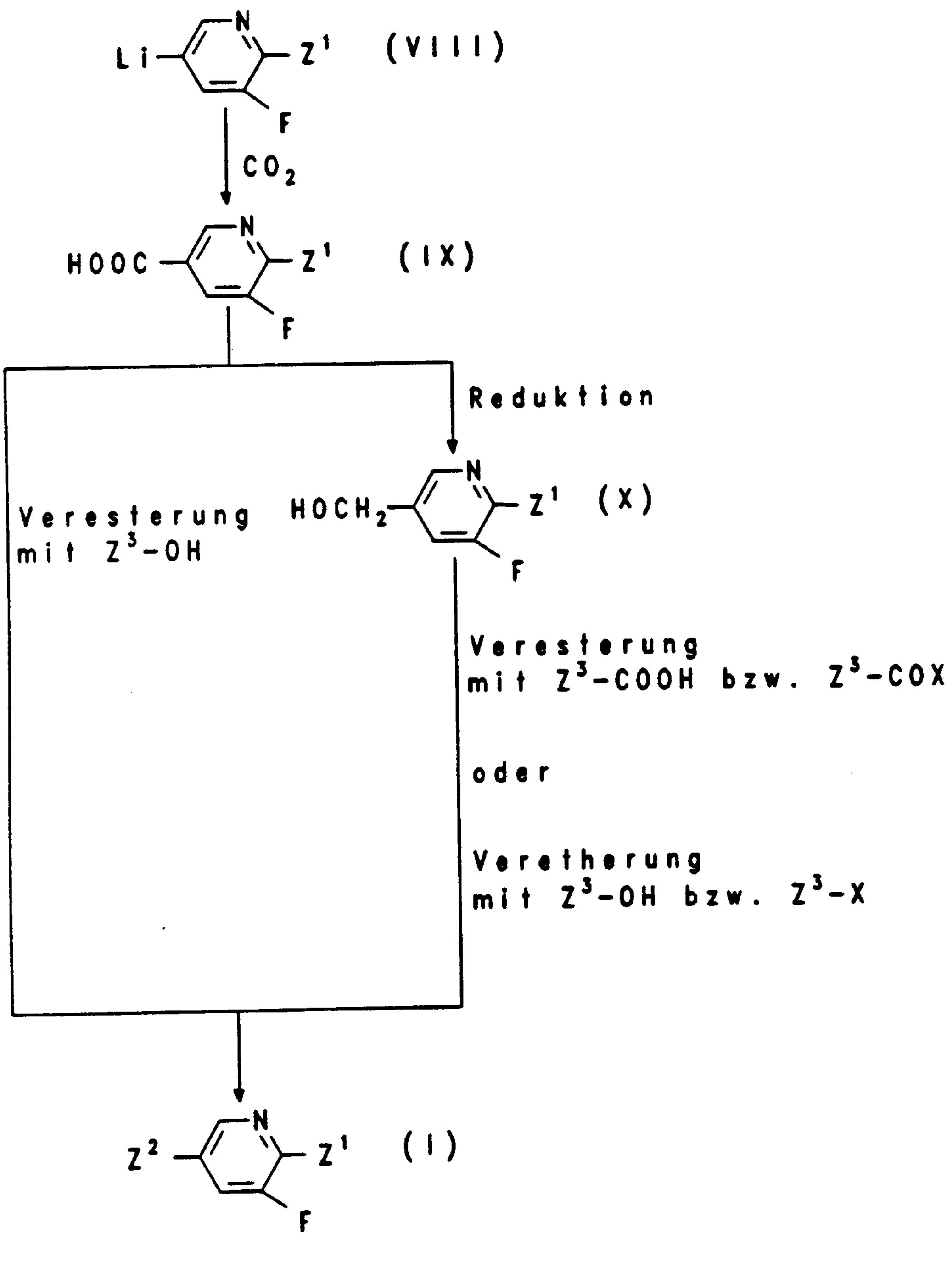


$Z^1$, $Z^2$ siehe Schema 2

$Z^3 = R^1 (-A^1)_k(-M^1)_l(-A^2)_m-$

X = Cl, Br, I

Schema 4:

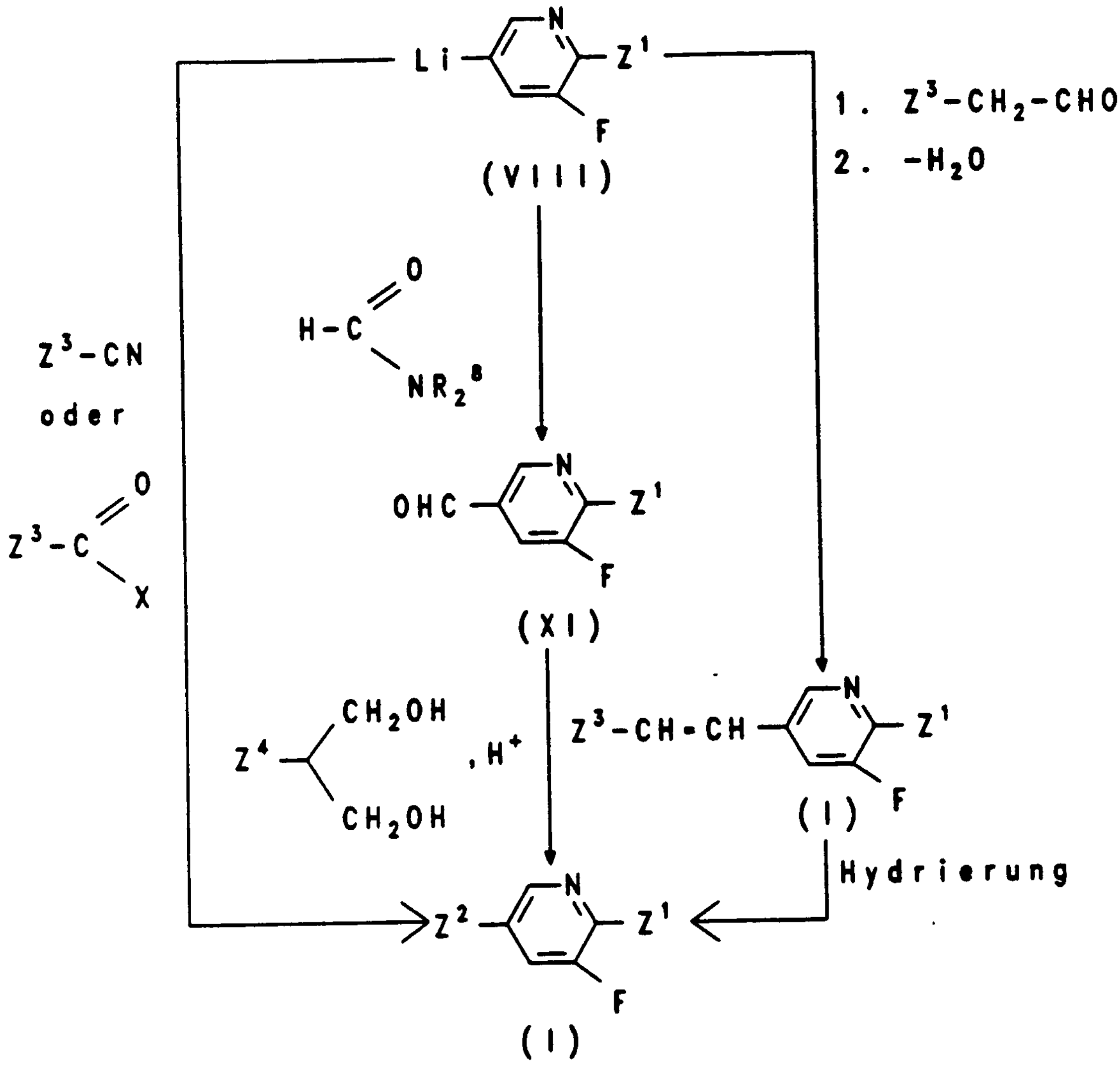


$Z^1$, $Z^2$, $Z^3$, X siehe Schema 2 und 3.
$Z^4 = R^1(-A^1)_k(-M^1)_l$
$R^8$ = geradkettiges oder verzweigtes Alkyl mit 1 bis 10 C-Atomen

Schema 5:

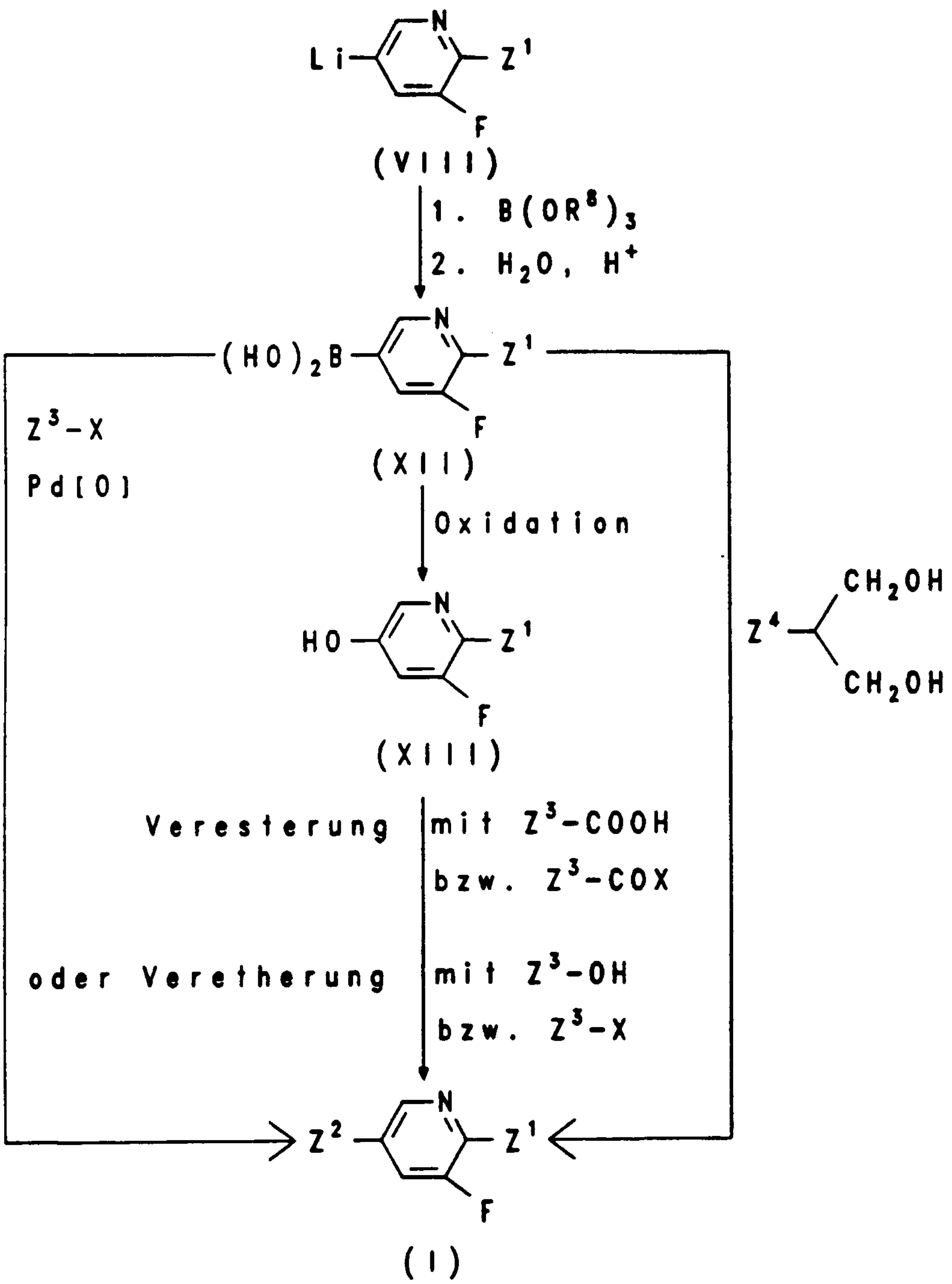


$Z^1$, $Z^2$, $Z^3$, $Z^4$, $R^8$, X siehe Schemata 2, 3 und 4.

Schema 6:

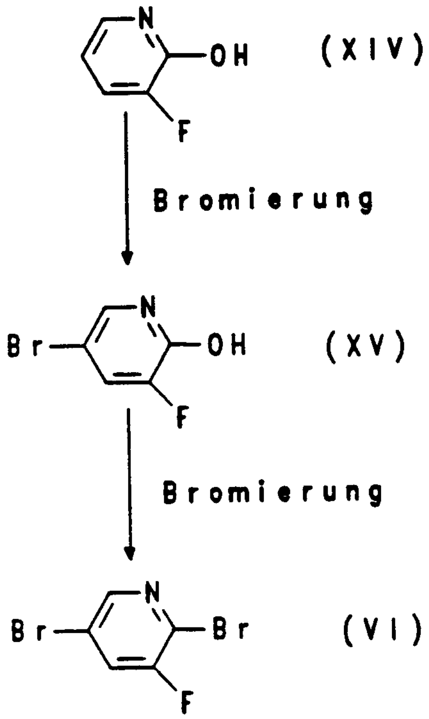


Die Erfindung wird durch die nachfolgenden Beispiele näher erläutert:

Beispiel 1: 3-Fluor-5-octyl-2-(4-octyloxyphenyl)pyridin

Zu 100 g (574,7 mmol) 2-Amino-5-Brompyridin in 300 ml konzentrierter Schwefelsäure (d = 1,84 g/$cm^3$) werden bei 60 °C 34,5 ml (821,6 mmol) rauchende Salpetersäure (d = 1,5 g/$cm^3$) getropft und anschließend 2 h bei 60 °C gerührt. Das Reaktionsgemisch wird auf Eiswasser gegossen, der ausgefallene Feststoff wird abfiltriert, mit Wasser gewaschen und getrocknet. Es werden 88,16 g 5-Brom-2-hydroxy-3-nitropyridin erhalten.

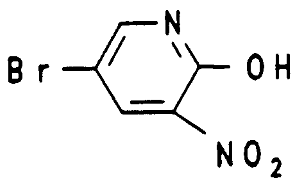

77,11 g (354,4 mmol) 5-Brom-2-hydroxy-3-nitropyridin werden zusammen mit 101,61 g (354,4 mmol) Phosphoroxytribromid und 33,7 ml (354,4 mmol) Phosphortribromid für 3 h auf 120 °C erhitzt. Anschließend wird das Reaktionsgemisch in kleinen Portionen vorsichtig auf Eiswasser gegossen, 1 h gerührt und dreimal mit Dichlormethan extrahiert. Die organische Phase wird zweimal mit Wasser gewaschen, über Natriumsulfat getrocknet, filtriert und vom

Lösungsmittel befreit. Nach chromatographischer Reinigung (Kieselgel/Dichlormethan) werden 43,05 g 2,5-Dibrom-3-nitropyridin erhalten.

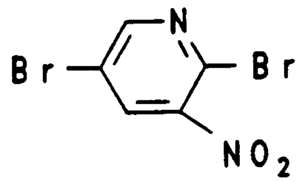

43,00 g (152,52 mmol) 2,5-Dibrom-3-nitropyridin werden unter Verwendung von 1,5 g Pd (10 %) auf Aktivkohle in 450 ml Methanol bis zum Verbrauch der berechneten Menge Wasserstoff hydriert, vom Katalysator abfiltriert und vom Lösungsmittel befreit. Es werden 37,56 g 3-Amino-2,5-dibrompyridin erhalten.

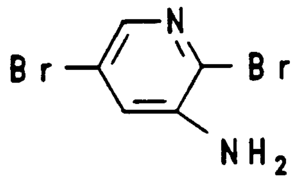

37,00 g (146,87 mmol) 3-Amino-2,5-dibrompyridin werden in 50 ml wäßriger $HBF_4$ (35 %ig) bei -10 °C mit 11,10 g (160,87 mmol) Natriumnitrit in 20 ml Wasser diazotiert. Nach halbstündigem Nachrühren bei -10 °C wird das Reaktionsgemisch 30 min auf 50 °C erhitzt, auf Eiswasser gegossen, mit Natriumhydrogencarbonat neutralisiert und dreimal mit Dichlormethan extrahiert. Die organische Phase wird zweimal mit Wasser gewaschen, über Natriumsulfat getrocknet, filtriert und vom Lösungsmittel befreit. Nach chromatographischer Reinigung (Kieselgel/Dichlormethan) werden 11,10 g 2,5-Dibrom-3-fluorpyridin erhalten.

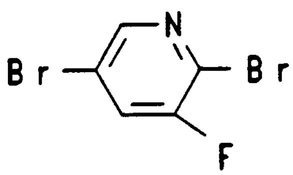

Aus 2,40 g (99,0 mmol) Magnesium und 25,56 g (89,6 mmol) 4-Octyloxybrombenzol in 250 ml Tetrahydrofuran werden in 3 h bei 60 °C die Lösung der Grignardverbindung hergestellt, welche zu einer auf -70 °C abgekühlten Lösung von 18,62 g (99,00 mmol) Triisopropylborat in 100 ml Tetrahydrofuran getropft und über Nacht gerührt wird. Anschließend werden 130 ml 10 %ige Salzsäure zugetropft und 1 h bei Raumtemperatur gerührt, zwischen Natriumchloridlösung und Ether verteilt, die organische Phase mit Natriumchloridlösung gewaschen, über Natriumsulfat getrocknet und eingeengt, wonach 21,26 g 4-Octyloxybenzolboronsäure erhalten werden.

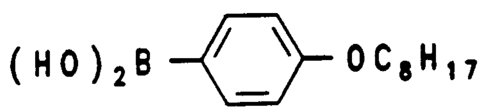

12,74 g (50,00 mmol) 2,5-Dibrom-3-fluorpyridin, 12,51 g (50,00 mmol) 4-Octyloxybenzolboronsäure, 0,58 g (0,50 mmol) Tetrakis(triphenylphosphin)palladium(0) und 10,60 g (100 mmol) Natriumcarbonat werden in 375 ml Toluol, 250 ml Ethanol und 125 ml Wasser 3 h auf 80 °C erhitzt. Anschließend wird zwischen wäßriger Natriumchloridlösung und

Ether verteilt, die organische Phase mit wäßriger Natriumchloridlösung gewaschen, über Natriumsulfat getrocknet, eingeengt und chromatographisch (Kieselgel/Hexan: Essigester = 9:1) gereinigt. Es werden 13,31 g 5-Brom-3-fluor-2-(4-octyloxyphenyl)pyridin erhalten.

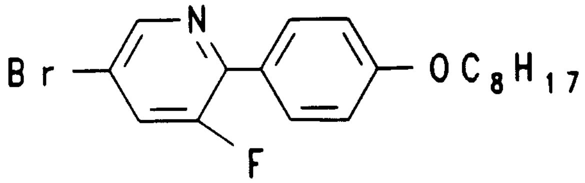

Aus 0,30 g (12,30 mmol) Magnesium und 2,16 g (11,18 mmol) Octylbromid in 10 ml Tetrahydrofuran wird in 2 h bei 50 °C die Lösung der Grignardverbindung hergestellt, welche zu einer auf -10 °C abgekühlten Lösung von 2,13 g (5,59 mmol) 5-Brom-3-fluor-2-(4-octyloxyphenyl)pyridin und 0,03 g (0,06 mmol) [1,3-Bis(diphenylphosphino)-propan]nickel(II)chlorid in 60 ml Tetrahydrofuran getropft und 3 h bei -10 °C gerührt wird. Anschließend wird zwischen Ether und wäßriger Ammoniumchloridlösung verteilt, die organische Phase zweimal mit wäßriger Natriumchloridlösung gewaschen, über Natriumsulfat getrocknet und zur Trockne eingeengt. Nach chromatographischer Reinigung (Kieselgel/Dichlormethan: Hexan 7:3) werden 1,89 g 3-Fluor-5-octyl-2-(4-octyloxyphenyl)pyridin erhalten.

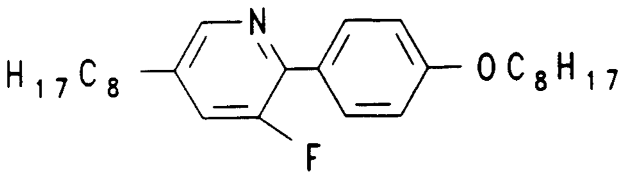

Die Verbindung zeigt die Phasenfolge:

X 25,5 (5) N 35 I.

Beispiel 2: 3-Fluor-5-octyloxy-2-(4-octyloxyphenyl)pyridin

Zu einer auf -70 °C abgekühlten Lösung von 7,61 g (20,00 mmol) 5-Brom-3-fluor-2-(4-octyloxyphenyl)pyridin in 400 ml Tetrahydrofuran werden 15 ml (24,00 mmol) 1,6 molare n-Butyllithiumlösung in Hexan getropft und 15 min bei -70 °C gerührt. Anschließend werden 7,5 g (40,00 mmol) Triisopropylborat zugetropft, 1,5 h bei -70 °C gerührt, auf Raumtemperatur erwärmt, 500 ml Ammoniumchloridlösung und 500 ml Ether zugegeben, die organische Phase abgetrennt und zweimal mit wäßriger Natriumchloridlösung gewaschen, über Natriumsulfat getrocknet und eingeengt. Die so erhaltene 3-Fluor-2-(4-octyloxyphenyl)pyridin-5-boronsäure wird in 100 ml Tetrahydrofuran mit 30 ml 17,5 Gew.-%iger wäßriger Wasserstoffperoxidlösung 2 h unter Rückfluß erhitzt, anschließend auf 0 °C abgekühlt und tropfenweise mit 200 ml wäßriger Natriumsulfitlösung versetzt. Die organische Phase wird abgetrennt, zweimal mit wäßriger Natriumchloridlösung gewaschen, über Natriumsulfat getrocknet und zur Trockne eingeengt. Nach Umkristallisation aus 1,2-Dichlorethan werden 5,08 g 3-Fluor-5-hydroxy-2-(4-octyloxyphenyl)pyridin erhalten.

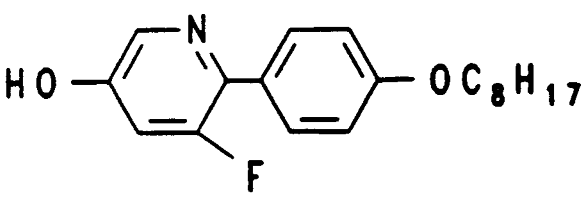

Zu 3,00 g (11,5 mmol) Triphenylphosphin in 50 ml Tetrahydrofuran werden bei 0 °C 2,00 g (11,5 mmol) Azodicarbonsäurediethylester getropft und 30 min. bei Raumtemperatur gerührt. Anschließend werden 2,42 g (7,7 mmol) 3-Fluor-5-hydroxy-2-(4-octyloxyphenyl)pyridin und 1,00 g (7,7 mmol) 1-Octanol zugegeben. Nach einer Reaktionszeit von 18 h bei Raumtemperatur wird das Lösungsmittel abdestilliert und der Rückstand chromatographisch (Kieselgel/Hexan: Ethylacetat 95:5) gereinigt. Nach Umkristallisation aus Acetonitril werden 1,38 g 3-Fluor-5-octyloxy-2-(4-octyloxyphenyl)pyridin erhalten.

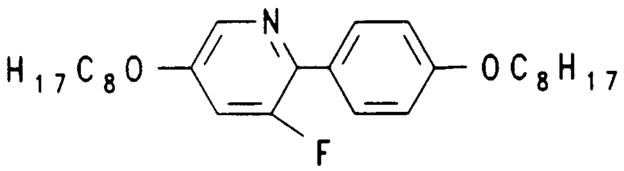

Die Verbindung zeigt die Phasenfolge:

X 51,3 (42,5) $S_A$ 50 N 69 I.

Beispiel 3: 5-Decyloxy-2-(4-decyloxyphenyl)-3-fluorpyridin

Die Synthese erfolgt analog Beispiel 2.

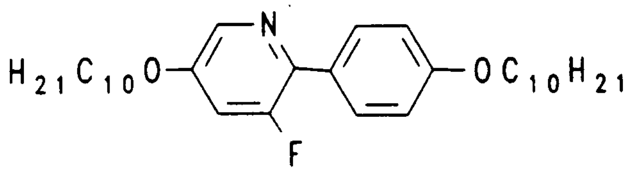

Die Verbindung zeigt die Phasenfolge:

X 57 (48) $S_c$ 49 $S_A$ 69 N 70 I

Beispiel 4: Octansäure-[3-fluor-2-(4-octyloxyphenyl)pyridin-5-yl]-ester

Zu 1,5 g (4,7 mmol) 3-Fluor-5-hydroxy-2-(4-octyloxyphenyl)pyridin in 20 ml Pyridin werden bei 0 °C 1,2 ml (7,1 mmol) Octansäurechlorid zugetropft und 3 h bei 0 °C gerührt. Anschließend wird auf Eiswasser gegossen, abfiltriert und der Rückstand chromatographisch (Kieselgel/Hexan: Ethylacetat 9:1) und durch Umkristallisation aus Acetonitril gereinigt. Es werden 1,34 g Octansäure-[3-fluor-2-(4-octyloxyphenyl)pyridin-5-yl]ester erhalten.

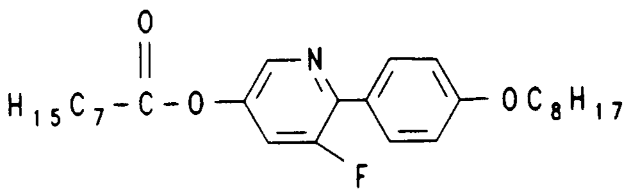

Beispiel 5: 3-Fluor-2-octyloxy-5-(4-octyloxyphenyl)pyridin

Zu 63,7 g (563,2 mmol) 3-Fluor-2-hydroxypyridin in 700 ml Dimethylformamid werden bei 0°C 32,0 ml (620,0 mmol) Brom getropft. Nach zweistündigem Nachrühren bei Raumtemperatur werden 800 ml Wasser zugegeben und 78 g $Na_2SO_3$ in 350 ml Wasser zugetropft. Anschließend wird dreimal mit je 400 ml Dichlormethan extrahiert, die organische Phase über $Na_2SO_4$ getrocknet und zur Trockne eingeengt. Es werden 97,6 g 5-Brom-3-fluor-2-hydroxypyridin erhalten.

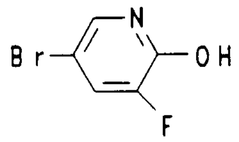

97,6 g (508,0 mmol) 5-Brom-3-fluor-2-hydroxypyridin werden in 500 ml Phosphortribromid 6 h bei 150°C gerührt. Anschließend wird auf Eiswasser gegossen, 2 h gerührt, dreimal mit Dichlormethan extrahiert, die organische Phase mit Natriumhydrogencarbonatlösung neutral gewaschen, über Natriumsulfat getrocknet und zur Trockne eingeengt. Nach chromatographischer Reinigung (Kieselgel/Dichlormethan) werden 76,53 g 2,5-Dibrom-3-fluorpyridin erhalten.

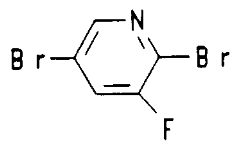

Lithiumoctanolat (das zuvor aus 13,02 g (100,00 mmol) 1-Octanol und 69 ml (110,00 mmol) einer 1,6 molaren n-Butyllithiumlösung in n-Hexan in 40 ml Tetrahydrofuran bei 0 °C hergestellt wurde) und 25,49 g (100,00 mmol) 2,5-Dibrom-3-fluorpyridin werden in 40 ml Tetrahydrofuran 7 h unter Rückfluß erhitzt. Anschließend wird zwischen wäßriger Natriumchloridlösung und Ether verteilt, die Etherphase zweimal mit wäßriger Natriumchloridlösung gewaschen, über Natriumsulfat getrocknet, filtriert und vom Lösungsmittel befreit. Nach chromatographischer Reinigung (Kieselgel/Hexan: Ethylacetat 9:1) werden 18,19 g (59,80 mmol) 5-Brom-3-fluor-2-octyloxypyridin erhalten.

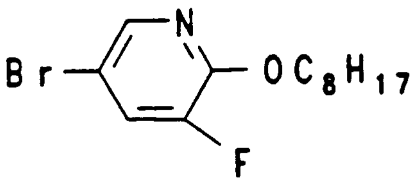

3,00 g (9,86 mmol) 5-Brom-3-fluor-2-octyloxypyridin, 2,47 g (9,86 mmol) 4-Octyloxybenzolboronsäure, 0,11 g (0,10 mmol) Tetrakis(triphenylphosphin)palladium(0) und 2,09 g (19,72 mmol) Natriumcarbonat werden in 90 ml Toluol, 60 ml Ethanol und 30 ml Wasser 3 h auf 80 °C erhitzt. Anschließend wird zwischen wäßriger Natriumchloridlösung und Ether verteilt, die organische Phase mit wäßriger Natriumchloridlösung gewaschen, über Natriumsulfat getrocknet, eingeengt und chromatographisch gereinigt (Kieselgel/Hexan: Ethylacetat 9:1). Es werden 2,85 g 3-Fluor-2-octyloxy-5-(4-octyloxyphenyl)pyridin erhalten.

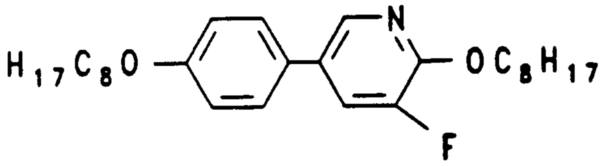

Beispiel 6: 3-Fluor-5-octyloxy-2-[4-(octyloxyphenyl)phenyl]pyridin

Die Darstellung erfolgt analog Beispiel 2

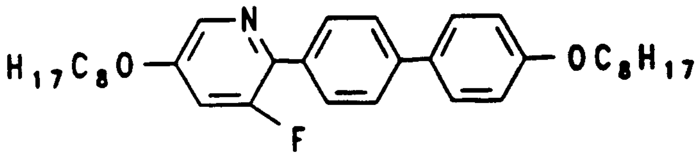

Beispiel 7: 3-Fluor-2,5-di(4-octyloxyphenyl)pyridin

4,00 g (10,51 mmol) 5-Brom-3-fluor-2-(4-octyloxyphenyl)pyridin, 2,63 g (10,51 mmol) 4-Octyloxybenzolboronsäure, 0,12 g (0,11 mmol) Tetrakis(triphenylphosphin)palladium(0) und 2,23 g (21,02 mmol) Natriumcarbonat werden in 100 ml Toluol, 70 ml Ethanol und 40 ml Wasser 3 h auf 80 °C erhitzt. Anschließend wird zwischen wäßriger Natriumchloridlösung und Ether verteilt, die organische Phase mit wäßriger Natriumchloridlösung gewaschen, über Natriumsulfat getrocknet, eingeengt und chromatographisch gereinigt (Kieselgel/Dichlormethan). Es werden 3,72 g 3-Fluor-2,5-di(4-octyloxyphenyl)pyridin erhalten.

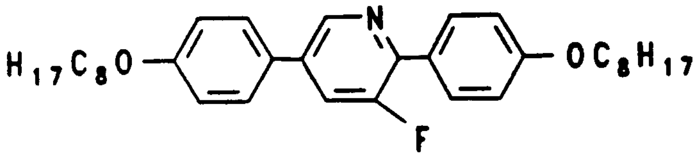

Die Verbindung zeigt die Phasenfolge:

X 88 (77,9) $S_c$ 142 $S_A$ 171 N 172 I.

Beispiel 8: 3-Fluor-2-octyloxy-5-[4-(4-octyloxyphenyl)phenyl]pyridin

Die Darstellung erfolgt analog Beispiel 5.

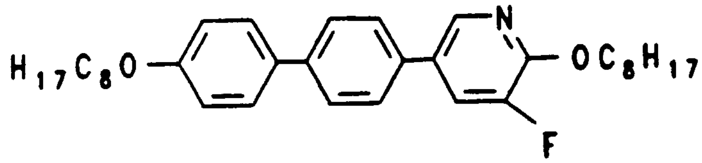

Beispiel 9: trans-4-Pentylcyclohexancarbonsäure-[3-fluor-2-(4-octyloxyphenyl)pyridin-5-yl]ester

0,29 g (0,91 mmol) 3-Fluor-5-hydroxy-2-(4-octyloxyphenyl)pyridin, 0,19 g (0,91 mmol) Dicyclohexylcarbodiimid, 0,16 g (0,91 mmol) trans-4-Pentylcyclohexancarbonsäure und 0,01 g 4-(N,N-Dimethylamino)pyridin werden in 10 ml Dichlormethan 18 h bei Raumtemperatur gerührt. Nach Filtration, Einengen zur Trockne, chromatographischer Reinigung (Kieselgel/Hexan: Ethylacetat 8:2) und Umkristallisation aus Acetonitril werden 0,19 g trans-4-Pentylcyclohexancarbonsäure-[3-fluor-2-(4-octyloxyphenyl)pyridin-5-yl]ester erhalten.

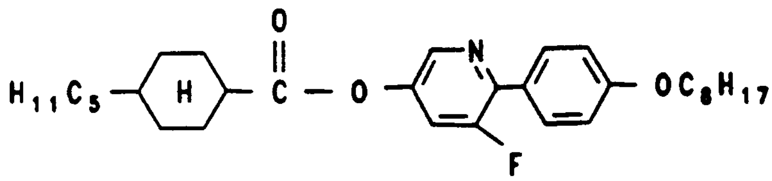

Die Verbindung zeigt die Phasenfolge:

X 65,2 (40,2) $S_c$ 63 $S_A$ 129 N 173 I.

Beispiel 10: trans-4-Pentylcyclohexancarbonsäure-[4-(3-fluor-5-octylpyridin-2-yl)phenyl]ester

Zu 35,24 g (130,0 mmol) tert.-Butyl-chlor-diphenylsilan und 11,25 g (65,0 mmol) 4-Bromphenol in 150 ml Dimethylformamid werden bei Raumtemperatur 11,06 g (162,5 mmol) Imidazol in 30 ml Dimethylformamid getropft. Nach einstündigem Rühren bei Raumtemperatur wird das Reaktionsgemisch auf 1000 ml 5 Gew.-%ige wäßrige Natriumhydrogencarbonatlösung gegossen, zweimal mit 400 ml Dichlormethan extrahiert, die organische Phase mit wäßriger Natriumchloridlösung gewaschen, über Natriumsulfat getrocknet, filtriert und zur Trockne eingeengt. Nach chromatographischer Reinigung (Kieselgel/Hexan: Ethylacetat 8:2) werden 23,40 g 4-Bromphenyl-tert.-butyl-diphenylsilylether erhalten.

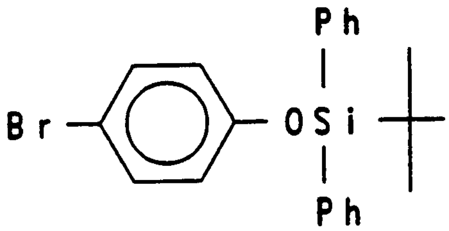

Analog Beispiel 1 wird aus 4-Bromphenyl-tert.-butyl-diphenylsilylether 4-tert.-Butyl-diphenylsilyloxybenzolboronsäure hergestellt.

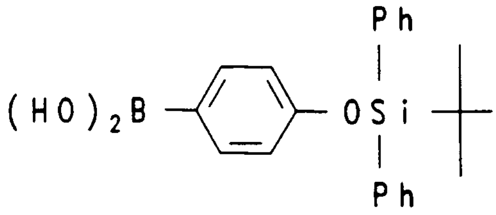

Analog Beispiel 1 wird aus 2,5-Dibrom-3-fluorpyridin und 4-tert.-Butyl-diphenylsilyloxybenzolboronsäure 5-Brom-2-(4-tert.-butyl-diphenylsilyloxyphenyl)-3-fluorpyridin hergestellt.

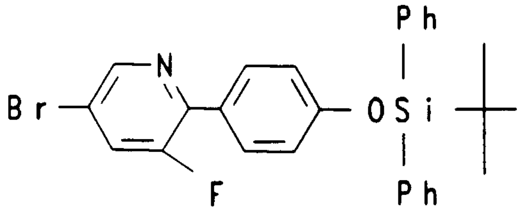

Analog Beispiel 2 wird aus 5-Brom-2-(4-tert.-butyl-diphenylsilyloxyphenyl)-3-fluorpyridin 2-(4-tert.-Butyl-diphenylsilyloxyphenyl)-3-fluor-5-hydroxypyridin hergestellt.

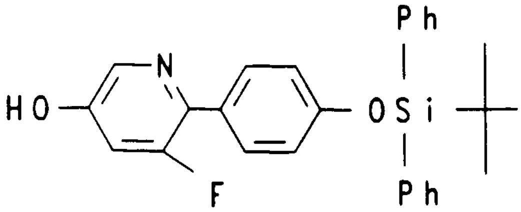

Analog Beispiel 2 wird aus 2-(4-tert-Butyl-diphenylsilyloxyphenyl)-3-fluor-5-hydroxypyridin und Octanol 2-(tert.-Butyl-diphenylsilyloxyphenyl)-3-fluor-5-octyloxypyridin hergestellt.

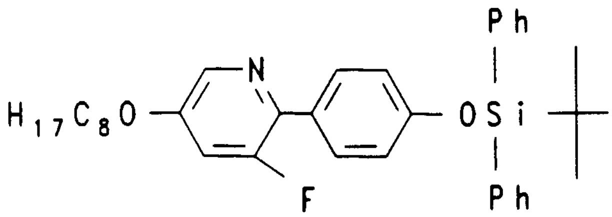

4,30 g (8,00 mmol) 2-(4-tert.-Butyl-diphenylsilyloxyphenyl)-3-fluor-5-octyloxypyridin werden mit 16 ml einer 1-molaren Tetrabutylammoniumfluoridlösung in Tetrahydrofuran in 50 ml Tetrahydrofuran 2 h bei Raumtemperatur gerührt. Anschließend wird mit wäßriger Natriumchloridlösung versetzt, mit Ether extrahiert, die Etherphase mit wäßriger Natriumchloridlösung gewaschen, über Natriumsulfat getrocknet, zur Trockne eingeengt und chromatographisch

(Kieselgel/Hexan: Ethylacetat 8:2) gereinigt. Es werden 2,16 g 3-Fluor-2-(4-hydroxyphenyl)-5-octyloxypyridin erhalten.

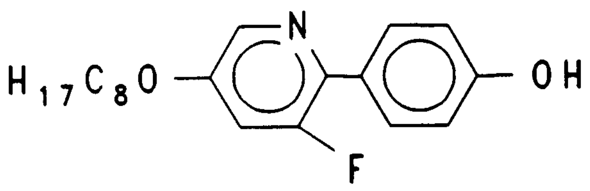

1,11 g (83,50 mmol) 3-Fluor-2-(4-hydroxyphenyl)-5-octyloxypyridin, 0,72 g (3,50 mmol) Dicyclohexylcarbodiimid, 0,69 g (3,50 mmol) trans-4-Pentylcyclohexancarbonsäure und 0,02 g 4-(N,N-Dimethylamino)pyridin werden in 20 ml Dichlormethan 3 h bei Raumtemperatur gerührt. Nach Filtration, Einengen zur Trockne, chromatographischer Reinigung (Kieselgel/Hexan: Essigester 8:2) und Umkristallisation aus n-Hexan werden 1,00 g trans-4-Pentylcyclohexancarbonsäure-[4-(3-fluor-5-octylpyridin-2-yl)phenyl]ester erhalten.

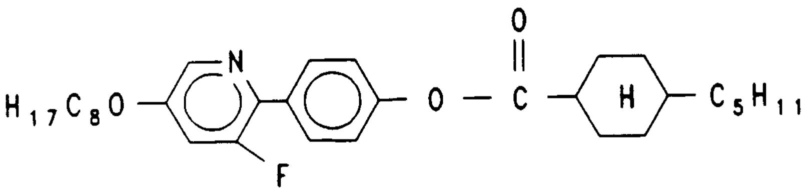

Die Verbindung zeigt die Phasenfolge: X 75 (60) N 169 I.

Beispiel 11: 3-Fluor-5-octyloxy-2-[4-(trans-4-pentylcyclohexyl)phenyl]pyridin

Analog Beispiel 1 wird aus 4-(Trans-4-pentylcyclohexyl)brombenzol 4-(Trans-4-pentylcyclohexyl)benzolboronsäure hergestellt.

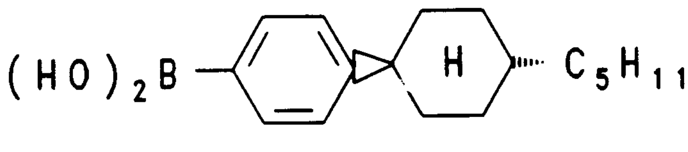

Analog Beispiel 1 wird aus 4-(Trans-4-pentylcyclohexyl)benzolboronsäure und 2,5-Dibrom-3-fluor-pyridin 5-Brom-3-fluor-2-[4-(trans-4-pentylcyclohexyl)phenyl]pyridin hergestellt.

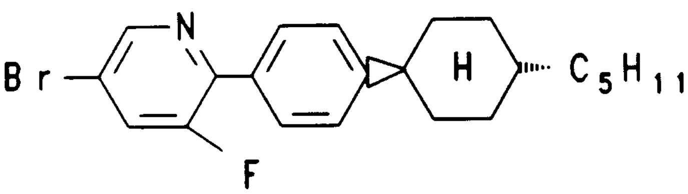

Analog Beispiel 2 erhält man aus 5-Brom-3-fluor-2-[4-(trans-4-pentylcyclohexyl)phenyl]pyridin 3-Fluor-5-hydroxy-2-[4-(trans-4-pentylcyclohexyl)phenyl]pyridin.

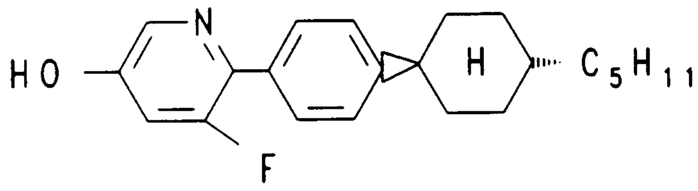

Analog Beispiel 2 wird aus Octanol und 3-Fluor-5-hydroxy-2-[4-(trans-4-pentylcyclohexyl)phenyl]pyridin 3-Fluor-5-ocryloxy-2-[4-(trans-4-pentylcyclohexyl)phenyl]pyridin erhalten.

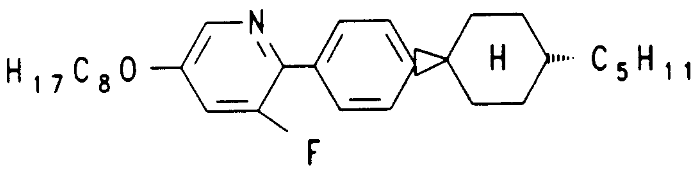

Beispiel 12: 3-Fluor-2-octyloxy-5-[4-(trans-4-pentylcyclohexyl)-phenyl]pyridin

Die Darstellung erfolgt analog Beispiel 5.

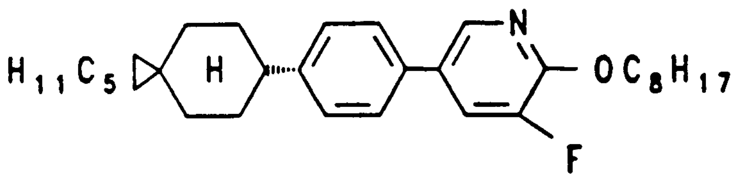

Beispiel 13: 3-Fluor-2-octyloxy-5-(6-octyloxynaphthalin-2-yl)-pyridin

Die Darstellung erfolgt analog Beispiel 5.

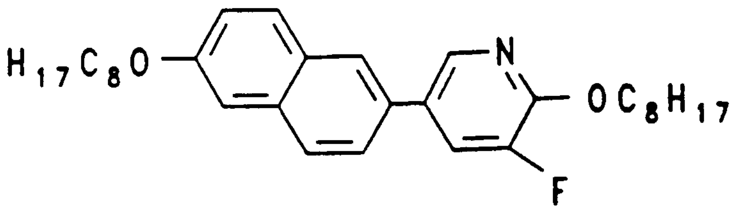

Beispiel 14: 3-Fluor-5-octyloxy-2-(6-octyloxynaphthalin-2-yl)pyridin

Die Darstellung erfolgt analog Beispiel 11.

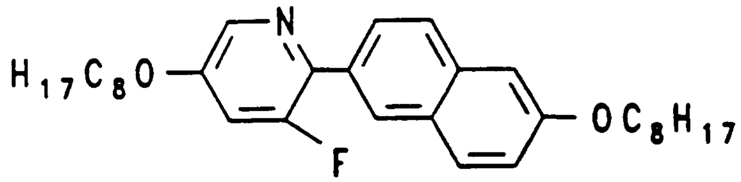

Beispiel 15: [(2S,3S)-3-Pentyloxiran-2-yl]methyl-[3-fluor-2-(4-octyloxyphenyl)pyridin-5-yl]ether

Zu 0,67 g (2,55 mmol) Triphenylphosphin in 15 ml Tetrahydrofuran werden bei 0 °C 0,44 g (2,55 mmol) Azodicarbonsäurediethylester getropft und 30 min. bei Raumtemperatur gerührt. Anschließend werden 0,54 g (1,70 mmol) 3-Fluor-5-hydroxy-2-(4-octyloxyphenyl)pyridin und 0,25 g (1,70 mmol) 2-[(2S,3S)-3-Pentyloxiranyl]methanol zugegeben. Nach einer Reaktionszeit von 18 h bei Raumtemperatur wird das Lösungsmittel abdestilliert und der Rückstand chromatographisch (Kieselgel/Hexan:Ethylacetat 8:2) gereinigt. Die Umkristallisation aus Hexan: Ethylacetat 8:2) ergibt 0,28 g [(2S,3S)-3-Pentyloxiran-2-yl]methyl-[3-fluor-2-(4-octyloxyphenyl)pyridin-5-yl]ether.

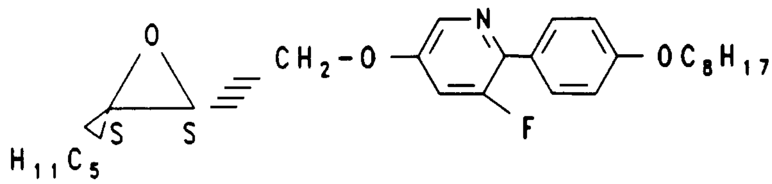

Die Verbindung zeigt die Phasenfolge:

X 54,6 (14,5) $S_c$ 53 $S_A$ 65 N 73 I.

Beispiel 16: [(2S,3S)-3-Pentyloxiran-2-yl]methyl-[4-(3-fluor-5-octyloxypyridin-2-yl)phenyl]ether

Zu 1,37 g (5,25 mmol) Triphenylphosphin in 20 ml Tetrahydrofuran werden bei 0 °C 0,91 g (5,25 mmol) Azodicarbonsäurediethylester getropft und 30 min bei 0 °C gerührt. Anschließend werden 1,11 g (3,50 mmol) 3-Fluor-2-(4-hydroxyphenyl)-5-octyloxypyridin und 0,75 g (5,25 mmol) 2-[(2S,3S)-3-Pentyloxiran-2-yl]methanol zugegeben. Nach einer Reaktionszeit von 18 h bei Raumtemperatur wird das Lösungsmittel abdestilliert und der Rückstand chromatographisch (Kieselgel/Hexan:Essigester 8:2) gereinigt. Die Umkristallisation aus Hexan ergibt 0,85 g [(2S,3S-3-Pentyloxiran-2-yl]methyl-[4-(3-fluor-5-octyloxypyridin-2-yl)phenyl]ether.

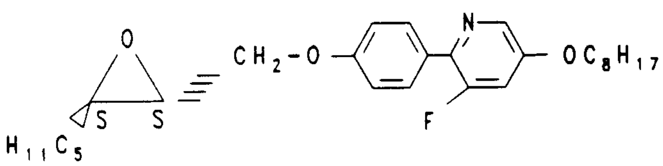

Die Verbindung zeigt die Phasenfolge:

X 55 (36) N 68 I.

Beispiel 17: [(2S,3S)-3-Pentyloxiran-2-yl]methyl-[3-fluor-2-(4-(trans-4-pentylcyclohexyl)phenyl)pyridin-5-yl]-ether

Die Darstellung erfolgt analog Beispiel 15.

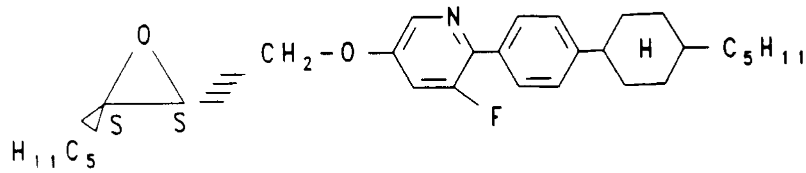

Anwendungsbeispiel 1:

a) Eine ferroelektrische Mischung, die aus den Komponenten

| | |
|---|---|
| 5-Octyloxy-2-(4-hexyloxyphenyl)pyrimidin | 22,8 Mol-% |
| 5-Octyloxy-2-(4-butyloxyphenyl)pyrimidin | 24,0 Mol-% |
| 5-Octyloxy-2-(4-decyloxyphenyl)pyrimidin | 19,2 Mol-% |
| 5-Octyloxy-2-(4-octyloxyphenyl)pyrimidin | 10,5 Mol-% |
| trans-4-Pentyl-cyclohexancarbonsäure-(4-(5-decylpyrimidin-2-yl))phenylester | 13,5 Mol-% |
| ((2S,3S)-3-Pentyloxiran-2-yl)methyl-(3-fluor-2-(4-octyloxyphenyl)pyridin-5-yl)ether | 10,0 Mol-% |

besteht, zeigt folgende flüssigkristalline Phasenbereiche:

X 3 $S^*_C$ 89 $S_A$ 94 N* 108 I

Sie weist bei einer Temperatur von 20 °C eine spontane Polarisation von 9,8 $nC/cm^2$ auf und schaltet bei einer Feldstärke von 10 V/µm mit einer Schaltzeit von 320 µs.

b) Im Vergleich dazu weist eine bekannte flüssigkristalline Mischung (DE 38 31 226.3), die sich von der obengenannten Mischung nur dadurch unterscheidet, daß sie keinen erfindungsgemäßen Dotierstoff enthält, folgende Phasenbereiche auf.

X 9 $S_C$ 84 $S_A$ 93 N 105 I

Diese Mischung belegt, daß mit den erfindungsgemäßen Verbindungen schnell schaltende ferroelektrische Mischungen herstellbar sind.

Anwendungsbeispiel 2:

a) Eine Mischung, die aus den Komponenten

| | |
|---|---|
| 5-Octyloxy-2-(4-hexyloxyphenyl)pyrimidin | 16,4 Mol-% |
| 5-Octyloxy-2-(4-decyloxyphenyl)pyrimidin | 10,9 Mol-% |
| 5-Decyl-2-(4-hexyloxyphenyl)pyrimidin | 10,6 Mol-% |
| 5-Octyl-2-(4-(7-cyclopropylheptyloxy)carbonyloxy-phenyl)-pyrimidin | 11,0 Mol-% |
| 5-(8-Cyclopropyloctyloxy)-2-(4-(4-transpentyl-cyclohexyl)-phenyl)-pyrimidin | 12,7 Mol-% |
| trans-4-Pentyl-cyclohexancarbonsäure-4-(5-(8-cyclopropyloctyl)-pyrimidin-2-yl)phenylester | 7,8 Mol-% |
| 5-(5-Cyclopropylpentyloxy-2-(4-hexyloxyphenyl)-pyrimidin | 11,7 Mol-% |
| ((2S,3S)-3-Pentyloxiran-2-yl)methyl-(4-(3-fluor-5-octyloxypyridin-2-yl)phenyl)ether | 10,0 Mol-% |

besteht, zeigt folgende flüssigkristalline Phasenbereiche:

X - 15 $S^*_C$ 75 $S_A$ 80 N* 91 I

Sie weist bei einer Temperatur von 25 °C eine spontane Polarisation von 3,5 nC/cm$^2$ auf.

b) Im Vergleich dazu weist die flüssigkristalline Mischung, die sich von der oben genannten Mischung nur dadurch unterscheidet, daß sie keinen erfindungsgemäßen Dotierstoff enthält, folgende Phasenbereiche auf.

X -13 $S_C$ 65 $S_A$ 70 N 86 I

Die Anwendungsbeispiele 1 und 2 belegen zudem, daß die Zugabe der erfindungsgemäßen Verbindungen zu einer Erhöhung des $S_C$-Phasenbereiches führt.

Anwendungsbeispiel 3

Eine Mischung besteht aus

30 Mol-% trans-4-Pentyl-cyclohexancarbonsäure-[3-fluor-2-(4-octyloxyphenyl)pyridin-5-yl]ester
Komponente A
70 Mol-% 4-Ethyl-2-fluoro-4-[2-(trans-4-n-pentylcyclohexyl)-ethyl]-biphenyl
Komponente B,

weist einen Klärpunkt von 135 °C auf und kristallisiert bei -28 °C.

Im Vergleich dazu weist die Komponente B folgende Phasenumwandlungen auf:

X 24 $S_B$ (13) N 103,4 I

Die Zugabe der erfindungsgemäßen Komponente A führt zu einer Erniedrigung des Kristallisationspunktes und zu einer Erhöhung des nematischen Phasenbereichs. Dieses Beispiel belegt, daß die erfindungsgemäßen Komponenten besonders gut geeignet sind, den nematischem Bereich von Flüssigkristallen zu verbreitern. Hervorzuheben ist ferner, daß die Verbindung in diesem Beispiel die dielektrische Anisotropie der Mischung verkleinert.

Anwendungsbeispiel 4

Eine achirale Grundmischung, die aus den Komponenten

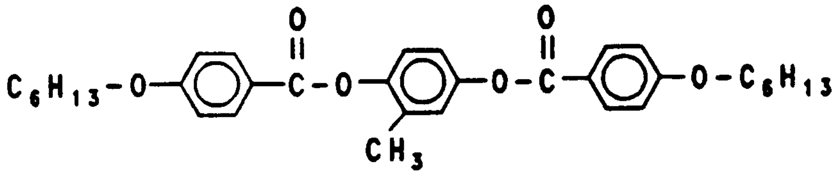

3,00 w.-%

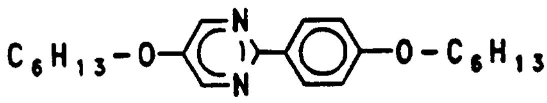

3,84 w.-%

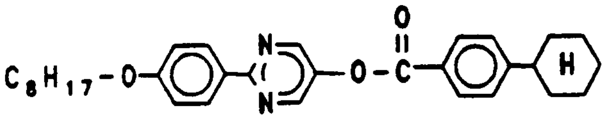

12,70 w.-%

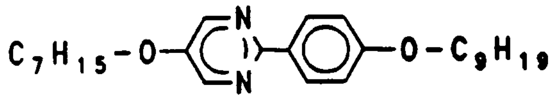

6,40 w.-%

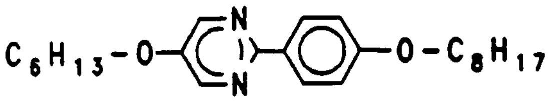

6,58 w.-%

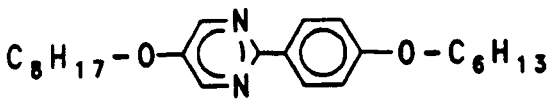

7,24 w.-%

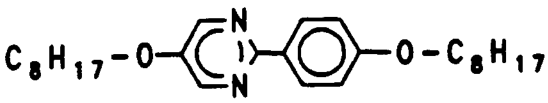

7,77 w.-%

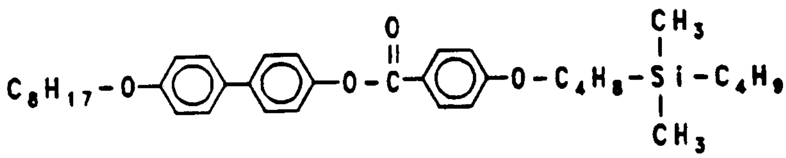

5,76 w.-%

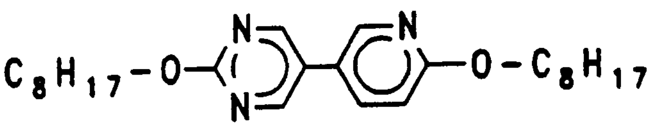

3,98 w.-%

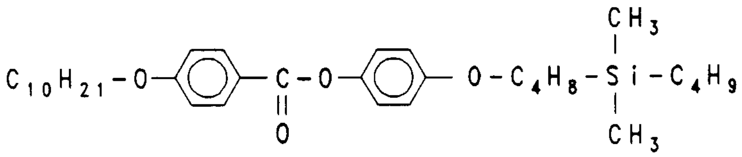

5,29 w.-%

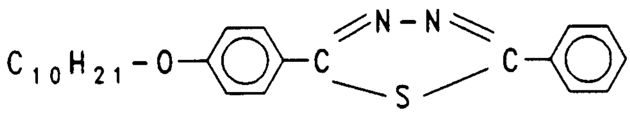

6,64 w.-%

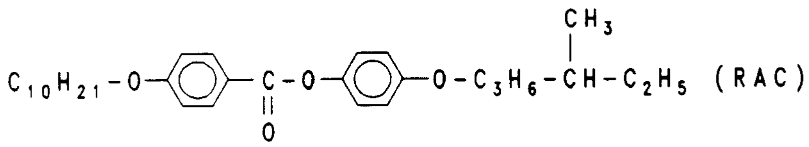

9,01 w.-%

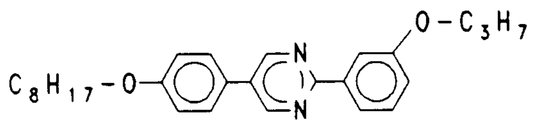

6,30 w.-%

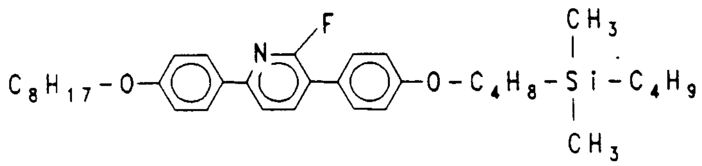

8,12 w.-%

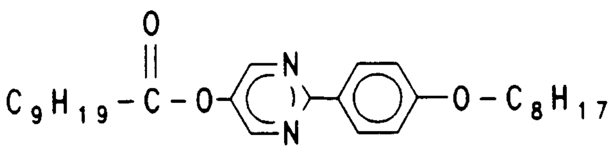

7,73 w.-%

besteht, zeigt folgende flüssigkristalline Phasenbereiche:

X -21 (-40) $S_c$ 79 $S_A$ 91 N 101 I

durch Zugabe von 10 w.-% der erfindungsgemäßen Substanz aus Beispiel 10 wird eine Mischung mit der Phasenfolge

X -22 (-45) $S_c$ 81 $S_A$ 85 N 106 I

erhalten.

Es zeigt sich, daß die erfindungsgemäße Substanz die nematische Phase verbreitert und den $S_A$-$S_c$-Übergang anhebt, gleichzeitig wird der Schmelzpunkt gesenkt. Eine Verbreiterung des Temperaturbereiches $S_c$-Phase sowohl zu hohen als auch niedrigen Temperaturen ist für die Anwendung von großem Vorteil, da hierdurch der Bereich der schaltbaren ferroelektrischen $S_c^*$ -Phase (bei Zusatz von chiralen Dotierstoffen) verbreitert wird.

Anwendungsbeispiel 5

Eine achirale Grundmischung, die aus den Komponenten

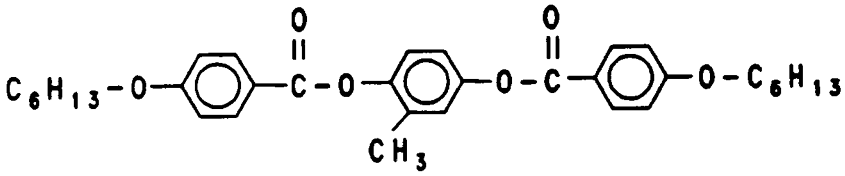

3,00 w.-%

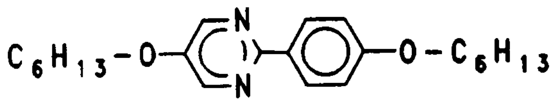

3,84 w.-%

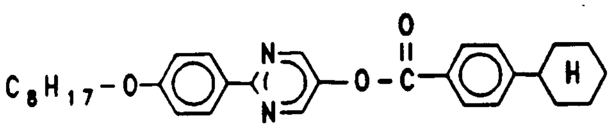

12,70 w.-%

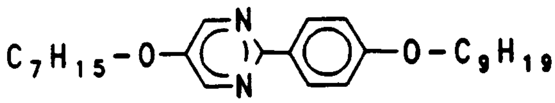

6,40 w.-%

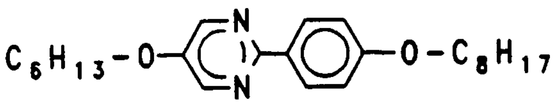

6,58 w.-%

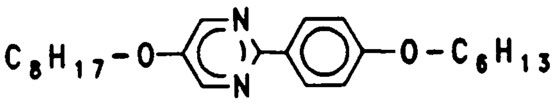

7,24 w.-%

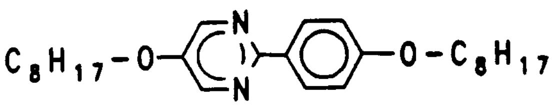

7,77 w.-%

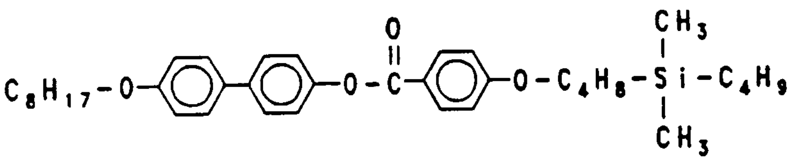

5,76 w.-%

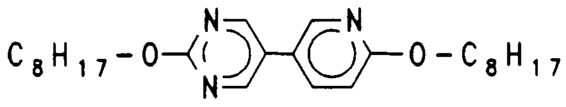

3,98 w.-%

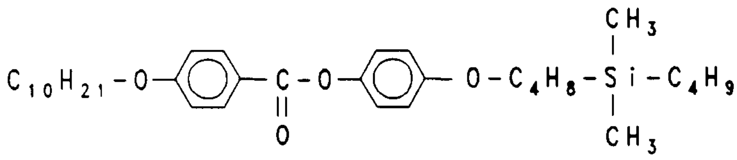

5,29 w.-%

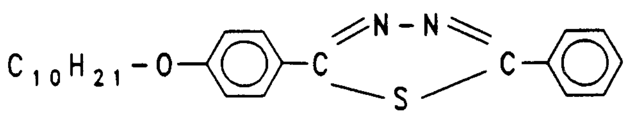

6,64 w.-%

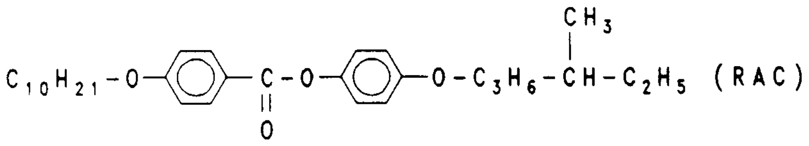

9,01 w.-%

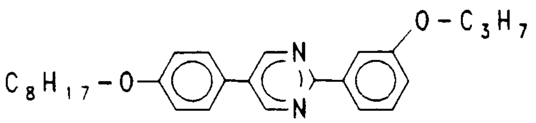

6,30 w.-%

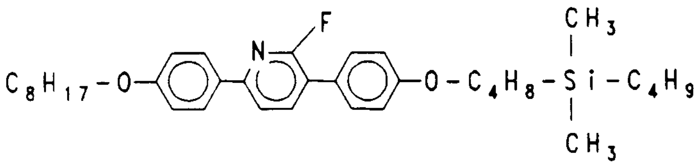

8,12 w.-%

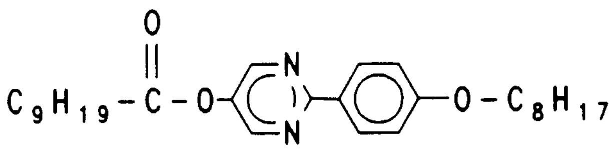

7,73 w.-%

besteht, zeigt folgende flüssigkristalline Phasenbereiche:

X -21 (-40) $S_c$ 79 $S_A$ 91 N 101 I

Durch Zugabe von 10 w.-% der erfindungsgemäßen Substanz aus Beispiel Nr. 16 wird eine ferroelektrische Mischung mit der Phasenfolge

X -35 (-41) $S_c$ 76 $S_A$ 85 N 98 I

erhalten.

Die chriale Substanz verändert die flüssigkristallinen Phasenübergänge kaum, während der Schmelzpunkt stark gesenkt wird.

Die spontane Polarisation wird zu

| T / °C | Ps / nC |
|---|---|
| 60 | 4,4 |
| 40 | 7,6 |
| 20 | 8,4 |

bestimmt.

Die erfindungsgemäße Substanz ermöglicht damit eine Variation der spontanen Polarisation und somit der Schaltgeschwindigkeit, ohne die LC-Phasen der Grundmischung stark zu verändern, bei gleichzeitigem Absenken des Schmelzpunktes.

**Patentansprüche**

**1.** 3-Fluorpyridinverbindung der allgemeinen Formel (I)

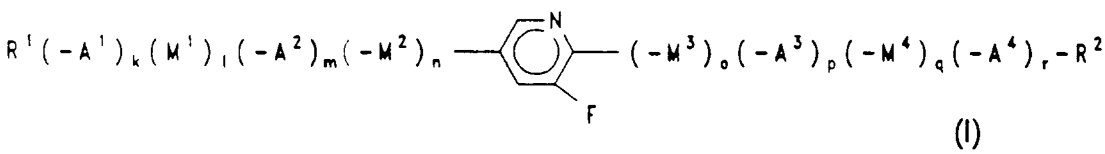

(I)

in der die Symbole folgende Bedeutung haben

$R^1$, $R^2$ gleich oder verschieden, -H, -F oder geradkettiges oder verzweigtes (mit oder ohne asymmetrisches C-Atom) Alkyl mit 1 bis 16 C-Atomen, wobei auch eine oder zwei nicht benachbarte $-CH_2$-Gruppen durch -O-,-CO-,-CO-O-,-O-CO-, -O-CO-O,

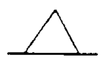

oder $-Si(CH_3)_2$- ersetzt sein können, und wobei auch ein oder mehrere H-Atome des Alkylrestes durch -F substituiert sein können, oder die nachfolgende chirale Gruppe:

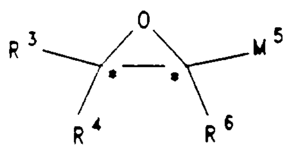

,

$A^1$, $A^2$, $A^3$, $A^4$ gleich oder verschieden, 1,4-Phenylen, Pyridin-2,5-diyl, Pyrimidin-2,5-diyl, wobei ein oder zwei H-Atome jeweils durch -F ersetzt sein können, trans-1,4-Cyclohexylen, Naphthalin-2,6-diyl,

$M^1$, $M^2$, $M^3$, $M^4$ gleich oder verschieden, -CO-O-, -O-CO-, $-CH_2-O-$, $-O-CH_2-$,

$R^3$, $R^4$, $R^6$, $R^7$ gleich oder verschieden, H oder geradkettiges oder verzweigtes Alkyl mit 1 bis 16 C-Atomen

$M^5$ $-CH_2-O-$, -CO-O-, $-O-CH_2-$, -O-CO-

k, l, m, n, o, p, q, r Null oder Eins, unter der Bedingung, daß die Summe k + m + p + r kleiner 4 und größer Null ist.

**2.** 3-Fluorpyridinverbindung nach Anspruch 1, dadurch gekennzeichnet, daß die Symbole in der allgemeinen Formel (I) folgende Bedeutung haben:

$R^1$, $R^2$ gleich oder verschieden, -H, -F oder geradkettiges oder verzweigtes (mit oder ohne asymmetrischem C-Atom) Alkyl mit 1 bis 16 C-Atomen, wobei auch eine $-CH_2$-Gruppe durch -O-, -CO-, -CO-O-, -O-CO-, -O-CO-O-,

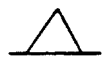

oder $-Si(CH_3)_2$- ersetzt sein kann, oder die nachfolgende chirale Gruppe:

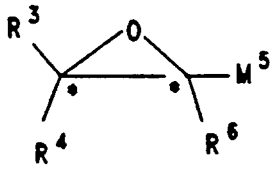

$A^1$, $A^2$, $A^3$, $A^4$ gleich oder verschieden, 1,4-Phenylen, Pyridin-2,5-diyl, Pyrimidin-2,5-diyl, wobei ein oder zwei H-Atome durch F ersetzt sein können, trans-1,4-Cyclohexylen, Naphthalin-2,6-diyl,

$M^1$, $M^2$, $M^3$, $M^4$ gleich oder verschieden, -CO-O-, -O-CO-, $CH_2O$; $-O-CH_2-$,

$R^3$, $R^4$, $R^6$, $R^7$ gleich oder verschieden, H oder geradkettiges oder verzweigtes Alkyl mit 1 bis 10 C-Atomen,

$M^5$ $-CH_2-O-$, CO-O-, $O-CH_2-$, -O-CO-.

**3.** Flüssigkristallmischung, enthaltend mindestens eine Verbindung der Formel I nach einem der Ansprüche 1 oder 2.

**4.** Flussigkristallmischung nach Anspruch 3, dadurch gekennzeichnet, daß die Flüssigkristallmischung ferroelektrisch ist.

**5.** Flüssigkristallmischung nach Anspruch 3, dadurch gekennzeichnet daß die Flüssigkristallmischung nematisch ist.

**6.** Schalt- und/oder Anzeigevorrichtung, enthaltend Trägerplatten, Elektroden, mindestens einen Polarisator, mindestens eine Orientierungsschicht sowie ein flüssigkristallines Medium, dadurch gekennzeichnet, daß das flüssigkristalline Medium eine Flüssigkristallmischung nach einem der Ansprüche 3 bis 5 ist.

**7.** Verfahren zur Herstellung eines 3-Fluorpyridin-Derivats nach Anspruch 1, dadurch gekennzeichnet, daß 2-Amino-5-brompyridin oder 3-Fluor-2-hydroxypyridin in mehreren Stufen zu 2,5-Dibrom-3-fluorpyridin umgesetzt wird und anschließend die beiden Bromatome in 2- und 5-Position in mehreren Stufen gegen die Ketten $R^1 (-A^1)_k(-M^1)_l(-A^2)_m(-M^2)_n$- bzw. $(-M^3)_o(-A^3)_n(-M^4)_q(-A^4)_r-R^2$ ausgetauscht werden. wobei die Symbole dieselbe Bedeutung wie in Anspruch 1 haben.

## Claims

**1.** 3-Fluoropyridine compound of the general formula (I)

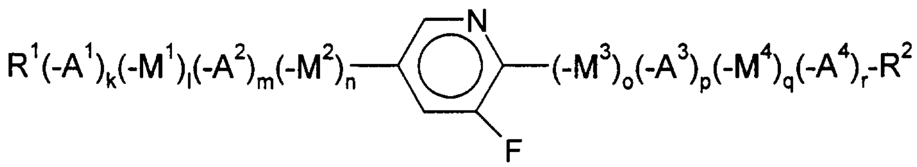

(I)

in which the symbols have the following meaning

$R^1$, $R^2$ the same or different, -H, -F or linear or branched (with or without asymmetric carbon atom) alkyl having 1 to 16 carbon atoms, wherein one or two non adjacent $-CH_2$-groups may be replaced by -O-, -CO-, -CO-O-, -O-CO-, -O-CO-O-,

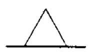

or $-Si-(CH_3)_2-$, and wherein one or more hydrogen atoms of the alkyl residue may be replaced by -F, or the following chiral group:

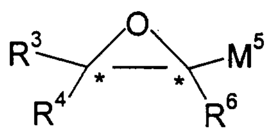

$A^1$, $A^2$, $A^3$, $A^4$ the same or different, 1,4-phenylene, pyridine-2,5-diyl, pyrimidine-2,5-diyl, wherein one or two hydrogen atoms may be replaced by F, trans-1,4-cyclohexylene, naphthalene-2,6-diyl;

$M^1$, $M^2$, $M^3$, $M^4$ the same or different, -CO-O-, -O-CO-, $-CH_2-O-$, $-O-CH_2-$;

$R^3$, $R^4$, $R^6$, $R^7$ the same or different, hydrogen or linear or branched alkyl having 1 to 16 carbon atoms;

$M^5$ $-CH_2-O-$, -CO-O-, -O-CH2-, -O-CO-;

k,l,m,n,o,p,q,r Zero or 1, with the condition that the sum k+m+p+r is less than 4 and more than Zero.

**2.** 3-Fluoropyridine compound according to claim 1, characterized in that the symbols in the general formula (I) have the following meanings:

$R^1$, $R^2$ the same or different, H, -F or linear or branched (with or without asymmetric carbon atom) alkyl having 1 to 16 carbon atoms, wherein one $-CH_2$-group may be replaced by -O-, -CO-, -CO-O-, -O-CO-, -O-CO-O-,

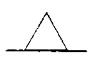

or $-Si(CH_3)_2-$, or the following chiral group:

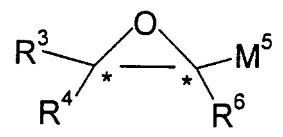

$A^1$, $A^2$, $A^3$, $A^4$ the same or different, 1,4-phenylene, pyridine-2,5-diyl, pyrimidine-2,5-diyl, wherein 1 or 2 hydrogen atoms may be replaced by F, trans-1,4-cyclohexylene, naphthaline-2,6-diyl;

$M^1$, $M^2$, $M^3$, $M^4$ the sane or different, -CO-O-, -O-CO-, $-CH_2-O-$, $-O-CH_2-$;

$R^3$, $R^4$, $R^6$, $R^7$ the sane or different, hydrogen or linear or branched alkyl having 1 to 10 carbon atoms;

$M^5$ $-CH_2-O-$, -CO-O-, $-O-CH_2-$, -O-CO-.

**3.** Liquid crystal mixture, containing at least one compound of the formula I according to claim 1 or 2.

**4.** Liquid crystal mixture according to claim 3, characterized in that the liquid crystal mixture is ferroelectric.

**5.** Liquid crystal mixture according to claim 3, characterized in that the liquid crystal mixture is nematic.

**6.** Switching and/or display means, containing supporting plates, electrodes, at least one polarizer, at least one orienting layer as well as a liquid crystalline medium, characterized in that the liquid crystalline medium is a liquid crystal mixture as defined in one of claims 3 to 5.

**7.** Process for preparing a 3-fluoropyridine derivative according to claim 1, characterized in that 2-amino-5-bromopyridine or 3-fluoro-2-hydroxypyridine is reacted in several steps to a 2,5-dibromo-3-fluoropyridine and subsequently the two bromine atoms in the 2- and 5-positions are replaced by the chains $R^1(-A^1)_k(-M^1)_l(-A^2)_m(-M^2)_n-$ and $(-M^3)_o(-A^3)_p(-M^4)_q(-A^4)_r(-R^2)$, respectively, in several steps, wherein the symbols have the same meaning as in claim 1.

**Revendications**

**1.** Composé de type 3-fluoropyridine de formule générale (I)

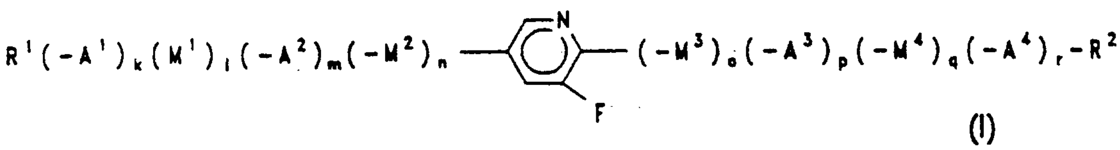

(I)

dans laquelle les symboles ont la signification suivante

$R^1$, $R^2$, identiques ou différents, -H, -F ou alkyle avec 1 à 16 atomes de C, rectiligne ou ramifié (avec ou sans atome de C asymétrique), un ou deux groupes $-CH_2-$ non voisins pouvant aussi être remplacés par -O-, -CO-, -CO-O-, -O-CO-, -O-CO-O-,

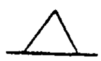

ou $-Si(CH_3)_2$, et un ou plusieurs atomes de H du radical alkyle pouvant aussi être substitués par -F ; ou le groupe chiral suivant :

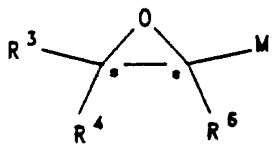

$A^1$, $A^2$, $A^3$, $A^4$, identiques ou différents, 1,4-phénylène, pyridine-2,5-diyle, pyrimidine-2,5-diyle, un ou deux atomes de H pouvant chacun être remplacé par -F ; trans-1,4-cyclohexylène, naphtalène-2,6-diyle ;
$M^1$, $M^2$, $M^3$, $M^4$, identiques ou différents, -CO-O-, -O-CO-, $-CH_2-O-$, $-OCH_2$ ;
$R^3$, $R^4$, $R^6$, $R^7$, identiques ou différents, H ou alkyle avec 1 à 16 atomes de C, rectiligne ou ramifié ;
$M^5$ $-CH_2-O-$, -CO-O-, $-O-CH_2-$, -O-CO- ;
k, l, n, o, p, q, r zéro ou un, à condition que la somme k+m+p+r soit inférieure à 4 et supérieure à zéro.

**2.** Composé de type 3-fluoropyridine selon la revendication 1, caractérisé en ce que les symboles dans la formule générale (I) ont la signification suivante :

$R^1$, $R^2$, identiques ou différents, -H, -F ou alkyle avec 1 à 16 atomes de C, rectiligne ou ramifié (avec ou sans atome de C asymétrique), un groupe $-CH_2-$ pouvant être aussi être remplacé par -O-, -CO-, -CO-O-, -O-CO-, -O-CO-O-,

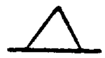

ou $-Si(CH_3)_2$ ; ou le groupe chiral suivant :

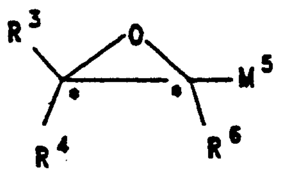

;

$A^1$, $A^2$, $A^3$, $A^4$, identiques ou différents, 1-4-phénylène, pyridine-2,5-diyle, pyrimidine-2,5-diyle, un ou deux atomes de H pouvant être remplacés par F ; trans-1,4-cyclohexylène, naphtalène-2,6-diyle ;
$M^1$, $M^2$, $M^3$, $M^4$, identiques ou différents, -CO-O-, -O-CO-, $-CH_2-O-$, $-OCH_2-$ ;
$R^3$, $R^4$, $R^6$, $R^7$, identiques ou différents, H ou alkyle avec 1 à 10 atomes de C, rectiligne ou ramifié ;
$M^5$ $-CH_2-O-$, -CO-O-, $-O-CH_2-$, -O-CO-.

**3.** Mélange pour cristal liquide contenant au moins un composé de formule I selon l'une des revendications 1 ou 2.

**4.** Mélange pour cristal liquide selon la revendication 3, caractérisé en ce que le mélange pour cristal liquide est ferroélectrique.

**5.** Mélange pour cristal liquide selon la revendication 3, caractérisé en ce que le mélange pour cristal liquide est nématique.

**6.** Dispositif de coupure de courant et/ou de signalisation contenant des plaques porteuses, des électrodes, au moins un polariseur, au moins une couche d'orientation, ainsi qu'un milieu de type cristal liquide, caractérisé en ce que le milieu de type cristal liquide est un mélange pour cristal liquide selon l'une des revendications 3 à 5.

**7.** Procédé pour la préparation d'un dérivé de type 3-fluoropyridine selon la revendication 1, caractérisé en ce que l'on transforme la 2-amino-5-bromopyridine ou la 3-fluoro-2-hydroxypyridine, en plusieurs étapes, en 2,5-dibromo-3-fluoropyridine et on échange ensuite les deux atomes de brome en positions 2 et 5, en plusieurs étapes, contre les

chaînes $R^1(-A^1)_k(M^1)_l(-A^2)_m(-M^2)_n$- respectivement $(-M^3)_o(-A^3)_p(-M^4)_q(-M^4)_r-R^2$, les symboles ayant la même signification que dans la revendication 1.